(19) **Europäisches Patentamt / European Patent Office / Office européen des brevets**

(11) **EP 4 447 797 B1**

(12) **EUROPEAN PATENT SPECIFICATION**

(45) Date of publication and mention of the grant of the patent:
**15.07.2026 Bulletin 2026/29**

(21) Application number: **22908541.0**

(22) Date of filing: **19.12.2022**

(51) International Patent Classification (IPC):
$A61B\ 5/00^{(2006.01)}$    $A61B\ 5/026^{(2006.01)}$
$G01J\ 3/02^{(2006.01)}$    $G01J\ 3/10^{(2006.01)}$
$G01J\ 3/44^{(2006.01)}$    $G01J\ 3/457^{(2006.01)}$
$G01J\ 1/44^{(2006.01)}$    $G01J\ 3/433^{(2006.01)}$
$G01N\ 21/47^{(2006.01)}$

(52) Cooperative Patent Classification (CPC):
**A61B 5/0261; A61B 5/0075; G01J 3/0218; G01J 3/10; G01J 3/433; G01J 3/4412;** G01J 2001/442; G01J 2001/4466; G01J 2003/4334

(86) International application number:
**PCT/US2022/053417**

(87) International publication number:
**WO 2023/114553 (22.06.2023 Gazette 2023/25)**

(54) **SYSTEMS, METHODS, AND MEDIA FOR FREQUENCY DOMAIN DIFFUSE CORRELATION SPECTROSCOPY**

SYSTEME, VERFAHREN UND MEDIEN FÜR DIFFUSE FREQUENZBEREICHSKORRELATIONSSPEKTROSKOPIE

SYSTÈMES, PROCÉDÉS, ET SUPPORT DE SPECTROSCOPIE DE CORRÉLATION DIFFUSE À DOMAINE FRÉQUENTIEL

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.12.2021 US 202163265626 P**

(43) Date of publication of application:
**23.10.2024 Bulletin 2024/43**

(73) Proprietor: **University Of South Florida**
**Tampa, FL 33612-9220 (US)**

(72) Inventors:
• **PARTHASARATHY, Ashwin**
  **Tampa, Florida 33612 (US)**
• **MOKA, Sadhu**
  **Tampa, Florida 33612 (US)**
• **SAFI, Abdul Mohaimen**
  **Tampa, Florida 33612 (US)**

(74) Representative: **J A Kemp LLP**
**80 Turnmill Street**
**London EC1M 5QU (GB)**

(56) References cited:
**US-A1- 2018 070 830    US-A1- 2019 000 321**
**US-A1- 2019 261 869    US-A1- 2019 261 869**
**US-A1- 2021 338 092**

• **APPLEGATE MATTHEW B. ET AL: "Modulation frequency selection and efficient look-up table inversion for frequency domain diffuse optical spectroscopy", JOURNAL OF BIOMEDICAL OPTICS, vol. 26, no. 03, 25 March 2021 (2021-03-25), 1000 20th St. Bellingham WA 98225-6705 USA, XP093326467, ISSN: 1083-3668, DOI: 10.1117/1.JBO.26.3.036007**

• MOKA SADHU ET AL: "Frequency domain diffuse correlation spectroscopy: a new method for simultaneous estimation of static and dynamic tissue optical properties", PROGRESS IN BIOMEDICAL OPTICS AND IMAGING, SPIE - INTERNATIONAL SOCIETY FOR OPTICAL ENGINEERING, BELLINGHAM, WA, US, vol. 11944, 4 March 2022 (2022-03-04), pages 1194409 - 1194409, XP060154909, ISSN: 1605-7422, ISBN: 978-1-5106-0027-0, DOI: 10.1117/12.2610115

**Description**

CROSS-REFERENCE TO RELATED APPLICATIONS

This application is based on, claims the benefit of, and claims priority to U.S. Provisional Patent Application No. 63/265,626 filed on December 17, 2021.

STATEMENT REGARDING FEDERALLY SPONSORED RESEARCH

N/A

BACKGROUND

Optical techniques based on the diffusion of light through tissue have been used to measure endogenous functional/physiological biomarkers of different tissue types/diseases from structures a few centimeters below the tissue surface. Due to a niche combination of clinical utility, measurement portability and safety, optical technologies have tremendous potential and power to be a ubiquitous clinical bedside monitor of tissue function.

Devices based on Diffuse Correlation Spectroscopy (DCS) and/or Diffuse Optical Spectroscopy (DOS) are described in US 2019/000321, US 2019/261869, and APPLEGATE MATTHEW B. ET AL: "Modulation frequency selection and efficient look-up table inversion for frequency domain diffuse optical spectroscopy",JOURNAL OF BIOMEDICAL OPTICS, 2021, XP093326467.

Accordingly, systems, methods, and media for frequency domain diffuse correlation spectroscopy are desirable.

SUMMARY

In accordance with some embodiments of the disclosed subject matter, systems, methods, and media for frequency domain diffuse correlation spectroscopy are provided.

In accordance with some embodiments of the disclosed subject matter, a system for frequency domain diffuse correlation spectroscopy is provided, the system comprising: an intensity modulated coherent light source; a photon counting detector; at least one hardware processor that is programmed to: cause the light source to emit light at a plurality of different intensity modulation frequencies toward a tissue sample; detect, for each of the plurality of different modulation frequencies, photon counts over a predetermined period of time; determine, for each of the plurality of modulation frequencies, a normalized intensity auto-correlation function; estimate a plurality of properties of the tissue sample using the plurality of intensity auto-correlation functions; and output at least one of the plurality of properties.

In some embodiments, the intensity modulated coherent light source comprises: a laser; and a signal generator.

In some embodiments, the photon counting detector comprises an avalanche photodiode.

In some embodiments, the plurality of different modulation frequencies comprises at least one frequency in a range of 50 megahertz (MHz) and 600 MHz.

In some embodiments, the intensity auto-correlation function is determined based on an equation expressed as:

$g_2(\tau) = \frac{\langle I(t)I(t+\tau)\rangle_t}{\langle I(t)\rangle^2}$ , where $I(t)$ is intensity at the photon counting detector at time t, and $I(t+\tau)$ is intensity at the photon counting detector at time $t + \tau$.

In some embodiments, the normalized intensity auto-correlation function is modeled based on an equation expressed as: $g_2(\tau, \rho, \omega) = 1 + \beta[(1 - m)|g_1(\rho, \tau, \omega = 0)|^2 + (m)|g_1(\rho, \tau, \omega = 0)|^2]$ where $\beta$ is a speckle averaging factor, $m$ is modulation depth, and $g_1(\rho, \tau, \omega = 0)$ is a normalized electric field auto-correlation function; and wherein the at least one hardware processor that is further programmed to: estimate at least a subset of the plurality of properties of the tissue sample using the plurality of intensity auto-correlation functions based on the modeling of the normalized intensity auto-correlation function.

In some embodiments, the normalized intensity auto-correlation function is modeled based on an equation expressed as:

$$g_{2_{FD}}(\rho, \tau, \omega) = 1 + \beta\left[\frac{|g_{1dc}(\tau)|^2 + 2m|g_{1dc}||g_{1ac}| + m^2|g_{1ac}(\tau)|^2}{(1+m)^2}\right]$$

where $\beta$ is a speckle averaging factor, $m$ is modulation depth, and $g_{1dc}(\tau)$ and $g_{1ac}(\tau)$ are normalized electric field auto-correlation functions; and wherein the at least one hardware processor that is further programmed to: estimate at least a subset of the plurality of properties of the tissue sample using the plurality of intensity auto-correlation functions based on the modeling of the normalized intensity auto-correlation function.

In some embodiments, the plurality of properties comprises: a tissue blood flow index $F$; an absorption coefficient $\mu_a$; and a scattering coefficient $\mu'_s$.

In some embodiments, the plurality of properties comprises one or more of: oxy-hemoglobin; deoxy-hemoglobin; tissue oxygen saturation; or tissue metabolism rate of oxygen.

In accordance with some embodiments of the disclosed subject matter, a method for frequency domain diffuse correlation spectroscopy, comprising: causing an intensity modulated coherent light source to emit light at a plurality of different intensity modulation frequencies toward a tissue sample; detecting, for each of the plurality of different modulation frequencies, photon counts over a predetermined period of time using a photon counting detector; determining, for each of the plurality of modulation frequencies, a normalized intensity auto-correlation function; estimating a plurality of properties of the tissue sample using the plurality of intensity auto-correlation functions; and outputting at least one of the plurality of properties.

In accordance with some embodiments of the disclosed subject matter, a non-transitory computer readable medium containing computer executable instructions that, when executed by a processor, cause the processor to perform a method for frequency domain diffuse correlation spectroscopy, the method comprising: causing an intensity modulated coherent light source to emit light at a plurality of different intensity modulation frequencies toward a tissue sample; detecting, for each of the plurality of different modulation frequencies, photon counts over a predetermined period of time using a photon counting detector; determining, for each of the plurality of modulation frequencies, a normalized intensity auto-correlation function; estimating a plurality of properties of the tissue sample using the plurality of intensity auto-correlation functions; and outputting at least one of the plurality of properties.

BRIEF DESCRIPTION OF THE DRAWINGS

Various objects, features, and advantages of the disclosed subject matter can be more fully appreciated with reference to the following detailed description of the disclosed subject matter when considered in connection with the following drawings, in which like reference numerals identify like elements.

FIG. 1 shows an example of various techniques that can be used to characterize a tissue sample, including frequency domain diffuse correlation spectroscopy techniques described herein in accordance with some embodiments of the disclosed subject matter.

FIG. 2 shows examples of absorption and scattering interactions of photons with issue chromophores in the near-infrared region of the electromagnetic spectrum.

FIG. 3 shows an example of a system for frequency domain diffuse correlation spectroscopy in accordance with some embodiments of the disclosed subject matter.

FIG. 4 shows examples of source and detector geometries that can be used in connection with mechanisms described herein for frequency domain diffuse correlation spectroscopy in accordance with some embodiments of the disclosed subject matter.

FIG. 5 shows an example of hardware that can be used to implement a system for frequency domain diffuse correlation spectroscopy in accordance with some embodiments of the disclosed subject matter.

FIG. 6 shows an example of frequency domain diffuse correlation spectroscopy in accordance with some embodiments of the disclosed subject matter.

FIG. 7 shows an example of different path lengths that photons emitted substantially simultaneously can take through a tissue sample which can result in phase differences between detected photons in a system for frequency domain diffuse correlation spectroscopy in accordance with some embodiments of the disclosed subject matter.

FIG. 8 shows an example of numerically calculated frequency dependent intensity auto-correlation function for various modulation frequencies and time lags in accordance with some embodiments of the disclosed subject matter.

FIG. 9 shows an example of a numerically calculated frequency dependent intensity auto-correlation function at zero frequency as a function of time lag in accordance with some embodiments of the disclosed subject matter.

FIG. 10 shows an example of a numerically calculated intercept of the frequency dependent intensity auto-correlation function as a function of modulation frequency in accordance with some embodiments of the disclosed subject matter.

FIG. 11 shows an example of a process for frequency domain diffuse correlation spectroscopy in accordance with some embodiments of the disclosed subject matter.

FIG. 12 shows an example Fabry-Perot plot of an intensity modulated laser source modulated at 300 megahertz.

FIG. 13 shows an example of measured frequency dependent intensity auto-correlation function using a system for frequency domain diffuse correlation spectroscopy implemented in accordance with some embodiments of the disclosed subject matter.

FIG. 14 shows an example plot of sensitivity to absorption changes in tissue simulating phantoms when absorption coefficient is varied and scattering coefficient is kept constant of a frequency domain diffuse correlation spectroscopy system implemented in accordance with some embodiments of the disclosed subject matter.

FIG. 15 shows an example plot of sensitivity to scattering changes in tissue simulating phantoms when absorption coefficient is varied and scattering coefficient is kept constant of a frequency domain diffuse correlation spectroscopy system implemented in accordance with some embodiments of the disclosed subject matter.

FIG. 16 shows an example plot of sensitivity to BFI changes in tissue simulating phantoms when absorption coefficient is varied and scattering coefficient is kept constant of a frequency domain diffuse correlation spectroscopy system implemented in accordance with some embodiments of the disclosed subject matter.

FIG. 17 shows an example plot of sensitivity to scattering changes in tissue simulating phantoms when scattering coefficient is varied and absorption coefficient is kept constant of a frequency domain diffuse correlation spectroscopy system implemented in accordance with some embodiments of the disclosed subject matter.

FIG. 18 shows an example plot of sensitivity to absorption changes in tissue simulating phantoms when scattering coefficient is varied and absorption coefficient is kept constant of a frequency domain diffuse correlation spectroscopy system implemented in accordance with some embodiments of the disclosed subject matter.

FIG. 19 shows an example plot of sensitivity to BFI changes in tissue simulating phantoms when scattering coefficient is varied and absorption coefficient is kept constant of a frequency domain diffuse correlation spectroscopy system implemented in accordance with some embodiments of the disclosed subject matter.

## DETAILED DESCRIPTION

In accordance with some embodiments of the disclosed subject matter, mechanisms (which can include systems, methods, and media) for frequency domain diffuse correlation spectroscopy are provided.

Recently, research has been ongoing into developing clinical optical monitoring utilizing two complementary technologies to quantitatively estimate tissue hemodynamics - Diffuse Correlation Spectroscopy (DCS) that measures tissue perfusion by analyzing the temporal fluctuations of highly coherent light that has scattered through tissue, and multi-distance Diffuse Optical Spectroscopy (DOS) that is used to estimate tissue oxygenation from the differential absorption of light at two or more wavelengths. Together, these hemodynamic measurements can be used to compute tissue metabolic rate of oxygen, an important measure of overall tissue health, function and pathology.

Such an approach to bedside optical monitoring can combine DCS and multi-distance DOS optical fibers into a hybrid probe, to attempt to simultaneously measure both static (oxygen concentration) and dynamic (blood flow) tissue properties. However, this simple combination of two technologies often results in a bulky probe that inhibits bedside use in critical care, and/or in spatially constrained measurement geometries. Additionally, the approach to use recordings from multiple source-detector locations ensures that the optical measurements are not uniformly sensitive to the same tissue volume, resulting in systematic estimation errors in heterogenous tissues and/or responses. In practice, current optical monitoring technologies cannot characterize hemodynamic effects of focal changes in tissue function such as spatially localized strokes/tumors or functional activation, with sufficient accuracy or fidelity.

Quantitative measurements of tissue function, and technologies that facilitate such measurements, can impact all aspects of biomedical research, from disease diagnosis and treatment monitoring, to physiological modeling of healthy and pathologic tissues. In particular, optical technologies have facilitated *in vivo* deep-tissue hemodynamic measurements of tissue physiology. For example, optical monitoring is generally noninvasive, safe, and easy to use. As another example, certain optical technologies can facilitate sensitivity to two important markers of tissue function - blood flow (which can generally be correlated with oxygen delivery) and oxygen saturation (which can generally be correlated with oxygen consumption). Often, the primary goal of these noninvasive measurements is to elicit the effect of focal changes in tissue function, such as to monitor hemodynamic changes due to localized strokes/brain injuries, to image/monitor the hemodynamics effects of localized tumors in the body, and to characterize effects of functional activation of specific neuronal circuits in the brain. However, the accuracy/fidelity of such focal hemodynamic measurements is confounded by conventional approaches that cannot measure both blood flow and oxygen saturation using a single sensor. For example, as described above, research has been ongoing into technologies that utilize optical detections/measurements from multiple spatial positions on the surface to measure both blood flow and oxygen saturation, which results in systemic biases.

In some embodiments, mechanisms described herein can be used to implement diffuse optical instruments that facilitate robust quantitative monitoring of focal changes in both tissue oxygen saturation and blood flow, with measurements from one source-detector pair.

The combination of tissue blood flow, hemoglobin concentrations, and oxygen saturations ($StO_2$) measured using optical techniques can yield important information about the tissue metabolic rate of oxygen, as well as overall tissue health, function and pathology. However, most studies that use optics for hemodynamic monitoring use either DOS or DCS, risking data misinterpretations. For example, DCS blood flow metrics could help ascertain if DOS measurements of increased deoxy hemoglobin are a result of increased oxygen metabolism, or are a result of reduced blood supply. On the other hand, quantitative estimation of tissue perfusion from "DCS only" data require assumptions of tissue optical properties. Simplistic attempts to address these biases involve combining DOS/DCS optics into a hybrid probe have resulted in increased cost (2X increase) and reduced instrument portability (e.g., 3X larger probes, limiting regular use in spatially constrained applications like neonates, spine, and critical care). A more significant concern is the fidelity of hybrid optical measurements. Even with collocated sources and detectors, inherent differences in measurement geometry leads to DOS and DCS instruments sampling different tissue volumes, resulting in errors in estimation of blood flow, tissue oxygen saturation or both. Differences in sampling volume are especially significant in realistic human tissues (e.g., skin) that are heterogeneous. For example, in cerebral measurements, scalp, skull and brain tissues have significantly different optical properties. In such an example, DCS and multi-distance DOS may sample different volume fractions of these tissues. These effects further confound errors of multi-distance measurements of tissue hemodynamics.

In conventional DCS, a highly coherent near infrared light source illuminates the tissue and diffuses through the tissue via multiple trajectories or path-lengths. The ensemble back-scattered light is collected at a detector position, and recorded using single photon counting detectors. As light diffuses through tissue, multiple scattering and absorption events occur, resulting in a change of light intensity at the detector. However, since DCS uses a coherent light source, scattering from moving particles in tissue (e.g., red blood cells), causes temporal and spatial fluctuations in the detected intensity. In conventional DCS, the auto-correlation function ($g_2(\tau)$) of these fluctuating intensities is fit to the solution of the Correlation Diffusion Equation to estimate tissue blood flow/perfusion. For example, fast blood flow results in a rapid decay of the auto-correlation function, while a slower flow results in slower decay of the auto-correlation function.

As described above, in practice, DCS and DOS are used separately in hemodynamic studies, leading to systematic measurement errors and unrealistic interpretations. For example, an increased de-oxy hemoglobin measurements from a "DOS only" sysem (without blood flow information) cannot be definitely interpreted as either increased oxygen metabolism or reduced blood supply (or some combination thereof). As another example, to estimate blood flow using DCS, static optical properties (e.g., absorption co-efficient $\mu_a$ and scattering coefficient $\mu_s$) are required for the inverse model of Correlation Diffusion Equation. It is a common practice to assume values for these optical properties are known for a nominal tissue type, however the tissue being measured may not correspond to the nominal tissue type.

As described above, attempts have been made to address these practical difficulties by simultaneous taking DOS and DCS measurements using a hybrid probe. However, this somewhat simplistic solution suffers from partial volume effects. Since DOS and DCS fibers are typically located at different positions on the probe, the difference in measurement geometry leads to sampling of different tissue volumes by the DOS and DCS instruments. This can result in systematic errors in estimation of tissue oxygen saturation, blood flow, or both. For example, in the case of *in-vivo* measurements that are heterogeneous in nature, differences in sampling volumes can produce significant measurement errors. Additionally, using a hybrid DOS and DCS instrument can significantly increase the cost (by a factor of 2) and increase the size (e.g., by a factor 3, leading to reduced portability) compared to a DOS-only or DCS-only approach. The increased size of such a hybrid probe can restrict its usage in spatially constrained areas, such as the spine, in neonates, and in the intrathoracic cavity. Recently, other techniques, such as Time domain DCS and Interferometric NIRS have been proposed to acquire static and dynamic optical properties simultaneously, typically with measurements at one source-detector separation. However, thee techniques require complex and expensive instrumentation, such as custom pulsed laser sources, expensive enhanced detectors, and synchronized detection.

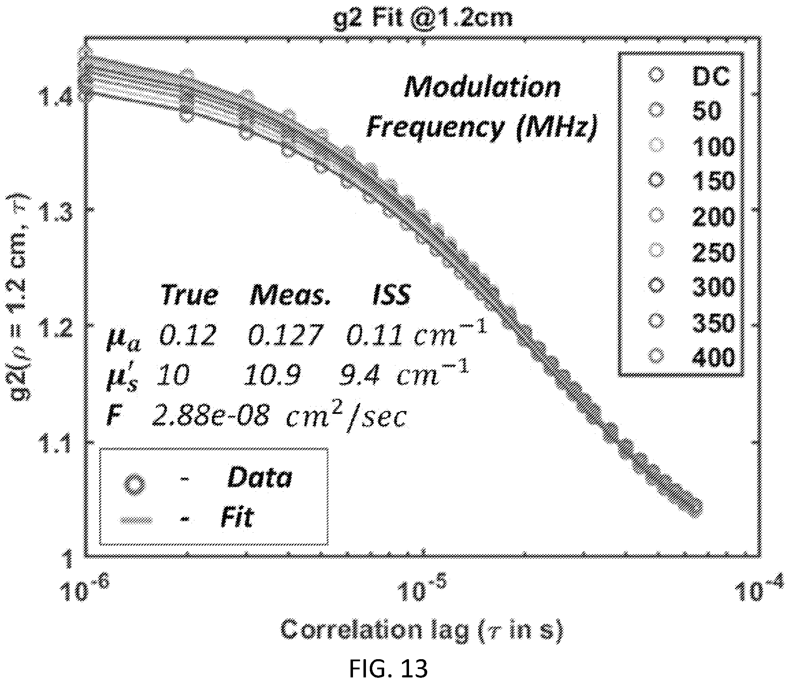

In some embodiments, mechanisms described herein can be used to implement a frequency domain DCS (FD-DCS) system that can be used to measure both static (oxygen concentration) and dynamic (blood flow) tissue properties. Results generated using such a device are described below in connection with FIGS. 13-19, and indicate that the FD-DCS mechanisms described herein can retrieve both static and dynamic optical properties of tissue. For example, in an experiment using a first phantom (phantom 1), absorption coefficient($\mu_a$) and scattering coefficient ($\mu_s$) are retrieved with a maximum error of 9%, while a commercial FD-DOS system retrieved the properties with maximum error of 10%. Additionally, in the example experiment, blood flow (F) was retrieved in the order of $1e - 8cm^2/sec$ with both an FD-DCS system implemented in accordance with mechanisms described and a conventional fast DCS system. The sensitivity of FD-DCS techniques described herein to absorption, scattering, and flow changes were verified, and associated experimental. Additionally, general diffusion models described below can include the effect of source modulation depth.

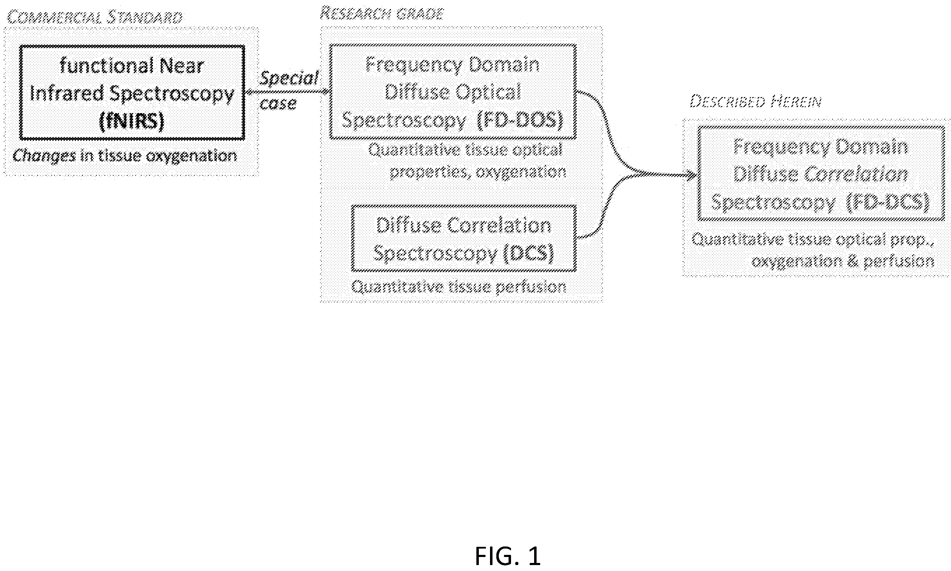

FIG. 1 shows an example of various techniques that can be used to characterize a tissue sample, including frequency domain diffuse correlation spectroscopy techniques described herein in accordance with some embodiments of the disclosed subject matter.

In some embodiments, mechanisms desceribed herein can be used to implement Frequency Domain Diffuse Correlation Spectroscopy (FD-DCS) techniques that can facilitate simultaneous acquisition of robust, quantitative measurements of tissue oxygen saturation and blood flow. For example, in FD-DCS, measurements at multiple modulation frequencies can be applied with DCS techniques to acquire $\mu_a$, $\mu_s$, and $F$ from intensity auto-correlation functions measured at a single source-detector separation.

In some embodiments, the intensity of a highly coherent laser light source can be modulated (e.g., at RF frequencies, such as $\omega$, 50 megahertz (*MHz*) - 400*MHz*) and a frequency dependent intensity auto-correlation function $g_2(\tau, \omega)$ can be measured. Additionally, in some embodiments, a new general light propagation model (which is sometimes referred to herein as Frequency Domain Correlation Diffusion Equation (FD-CDE) is described below, and a multi-parametric fit of the measured frequency dependent auto-correlation function to the solution of frequency domain CDE can facilitate simultaneous estimation of $\mu_a$, $\mu_s$ and $F$.

In some embodiments, FD-DCS can facilitae simultaneous measurement of both static and optical properties at a single source-detector separation, which can eliminate systematic errors caused due to geometric variation in multi-distance measurements. For example, multi-frequency measurements of an optical signal at a single source-detector separation can be expected to have similar tissue sensitivities. Additionally, it has been shown that tissue sampling volumes are relatively constant with modulation frequencies, for frequencies less than 600 MHz. In some embodiments, FD-DCS technques described herein can eliminate the need for multiple collocated sources and/or phase sensitive detectors, thus making the system portable and low cost. Additionally, in some emboediments, relatively high data acquisition rates can be achieved using FD-DCS (e.g., higher than rates that can be achieved by a hybrid DOS and DCS system) as the flow and oxygenation information is implicit in the data set received at the FD-DCS detector. Such advantages can make an FD-DCS system a solution for some of the issues in traditional DOS and DCS systems. Furthermore, FD-DCS can be implemented by replacing the source of a conventional fast DCS system with an intensity modulated coherent laser and using analysis techniques described herein, while the detector device can remain the same (e.g., decreasing the build time and cost).

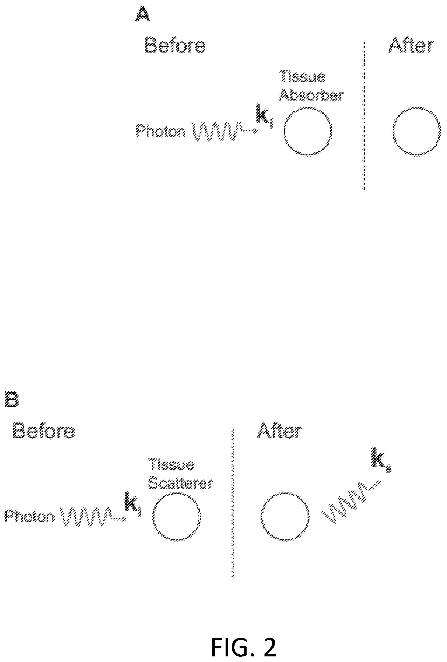

FIG. 2 shows examples of absorption and scattering interactions of photons with issue chromophores in the near-infrared region of the electromagnetic spectrum. As shown in FIG. 2, propagation of light through tissue in the NIR region can be characterized by two processes - absorption and scattering.

Absorption can refer to the loss of energy by a photon as it propagates/diffuses through tissue. Typically, this energy is converted to heat, but it can also lead to effects such as fluorescence. The light attenuation in tissue can be described by an absorption coefficient ($\mu_a$). The value $1/\mu_a$ can be referred to as the absorption mean free path, which can charaterize an average distance traveled by a photon in the tissue before it is absorbed. Absorption coefficient is also linearly related to the tissue chromophore concentration. Considering oxy-hemoglobin and de-oxy hemoglobin are the strongest absorbing endogenous tissue chromophores, tissue absorption coefficient can be expressed as: $\mu_a = \varepsilon_{HbO}C_{HbO} + \varepsilon_{HbR}C_{HbR}$, $C_{HbO}$ and $C_{HBR}$ are the concentrations of oxygenated and de-oxygenated hemoglobin (units of micromoles ($\mu M$)), $\varepsilon_{Hbo}$ and $\varepsilon_{HbR}$ are the corresponding molar extinction coefficients ($cm^{-1}/(moles/liter)$). Typical values of $\mu_a$ for tissue at near-infrared (NIR) wavelengths are around 0.02 - 0.3 $cm^{-1}$. In some embodiments, multi-wavelength tissue absorption measurements can facilitate allow measurement of chromophore concentration by using the spectra of these chromophores.

Scattering can refer to the deviation in the light path of a photon as it interacts with tissue. This deviation can occur due to refraction of light through microscopic refractive index changes in the tissue. Biological tissues vary in the refractive index spatially in the order of the NIR light wavelengths, which can facilitae probing of structural information of tissue molecules and chromophores. The heterogeneous regions of tissue can make the tissue a highly scattering medium at NIR wavelengths allowing to probe relatively deep tissue lengths.

The distance traveled by a photon before it is scattered in the tissue can be referred to as the scattering mean free path (which can be represented as $1/\mu_s$), and its reciprocal is the tissue scattering coefficient ($\mu_s$). Typical values of $\mu_s$ for NIR wavelengths are around 100 - 1000 $cm^{-1}$. Tissue scattering can be elastic (e.g., Rayleigh and Mie scattering) or inelastic (e.g., Raman scattering). Elastic scattering can refer to scattering of photons where light energy is conserved, while the incident light energy is different than the scattered light energy in the case of inelastic scattering. While Raman scattering can be used to probe tissue molecular properties, most scattering in tissue is elastic. Note that techniques decribed herein generally deal with diffusion of light in tissue using relatively low illumination intensities, and in this regime, scattering can be assumed to generally be elastic. A combination of absorption coefficient and scattering coefficient can be referred to as together can be referred to as the total attenuation coefficient ($\mu_t$), which can be expressed as:

$$\mu_t = \mu_a + \mu_s. \tag{1}$$

The inverse of the total attenuation coefficient can be referred to as the Mean Free Path (MFP). For NIR wavelengths, scattering coefficients are generally higher than absorption coefficient as scattering is more dominant in the NIR region of the electromagnetic spectrum.

Note that although the scattering coefficient gives information about the scattering in the tissue, it does not include information of the direction of the scattering. The information about the direction of scattering can be obtained by scattering anisotropy factor ($g$) which can be expressed as $g = <cos\theta>$, a measure of the average scattering angle. $g$ varies from -1 to 1. Positive values of $g$ can denote forward scattering and negative values of $g$ can indicate back scattering. Scattering is isotropic when $g = 0$. Tissue is mostly forward scattering with $g = 0.9$. Formal expressions for the anisotropy factor can be obtained from the Heyney-Greenstein phase function.

The usage of the scattering phase function can faciliate a derivation of the length that a photon travels before the direction is randomized and knowledge of its initial direction is lost. This can be referrd to as reduced photon scattering length, random-walk step length, or transport mean free path. The inverse of the transport mean free path is the reduced scattering coefficient $\mu'_s$, which can be expressed as:

$$\mu'_s = \mu_s(1 - g). \qquad (2)$$

A typical values of reduced scattering coefficient $\mu'_s$ in tissue is around 10 $cm^{-1}$. The transport attenuation coefficient ($\mu_{tr}$) which can be a better indicator of tissue penetration depths than the total attenuation coefficient in biological tissues that are highly anisotropic scattering medium can be expressed as:

$$\mu_{tr} = \mu_a + \mu_{s'}. \qquad (3)$$

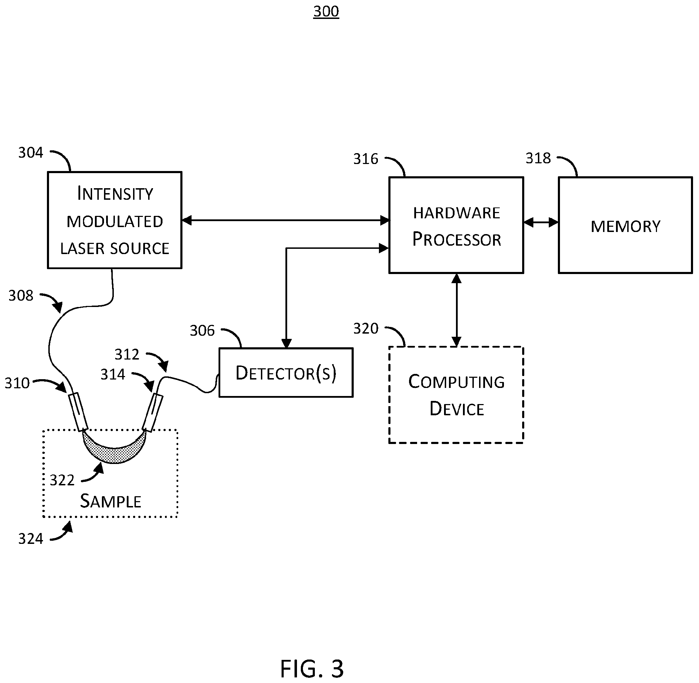

FIG. 3 shows an example 300 of a system for frequency domain diffuse correlation spectroscopy in accordance with some embodiments of the disclosed subject matter.

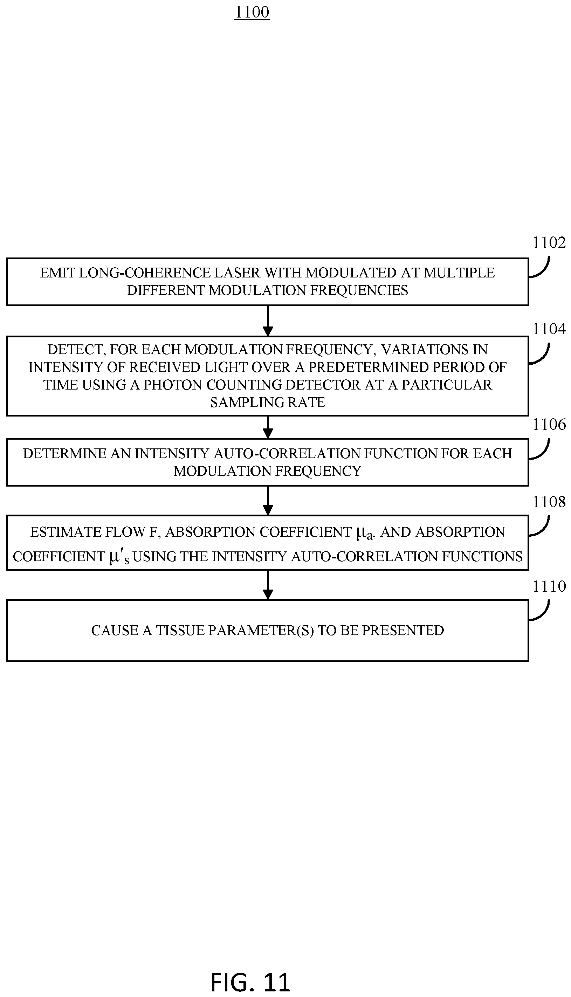

As shown, system 300 can include an intensity modulated light source 304; one or more detectors 306; a source waveguide 308 and a source waveguide positioning device 310; a detector waveguide 312 and a detector waveguide positioning device 314; hardware processor 316 configured to control operations of system 300 which can include any suitable hardware processor (such as a microprocessor, a central processing unit (CPU), an accelerated processing unit (APU), a graphics processing unit (GPU), a field programmable gate array (FPGA), an application specific integrated circuit (ASIC), a digital signal processor, a microcontroller, etc.) or combination of hardware processors; memory 318; and an external computing device 320 (e.g., which can be, for example, smartphone, a wearable computer, a tablet computer, a laptop computer, a personal computer, a game console, a server, etc., and can including a hardware processor or combination of hardware processors, a display, input device(s), a communication system(s), etc.). Although not shown, system 300 can include an input device (such as one or more buttons, a microphone, a touchscreen, etc.) for accepting input from a user and/or from the environment; one or more signal generators; a communication system or systems for allowing communication between hardware processor 316 and other devices, such as computing device 320 and/or other computing devices, via a communication link(s), and communication network, etc. In some embodiments, memory 318 can store raw data output by detectors 306, intensity auto-correlation functions, etc. Memory 318 can include a storage device (e.g., a hard disk, a Blu-ray disc, a Digital Video Disk, RAM, ROM, EEPROM, etc.) for storing a computer program for controlling hardware processor 316. In some embodiments, memory 318 can include instructions for causing hardware processor 316 to execute processes associated with mechanisms described herein, such as processes described below in connection with FIG. 11.

In some embodiments, source optical waveguide 308 can be any suitable optical fiber, such as one or more multi-mode fibers (e.g., having a diameter in a range of 400 micrometers ($\mu$m) to 1,000 $\mu$m). As another example, source optical waveguide 308 can be implemented using one or more single mode fibers. As yet another example, source optical waveguide 308 can be implemented using one or more few-mode fibers. In some embodiments, source optical waveguide 308 can be omitted. For example, a beam emitted from modulated light source 304 can be directed toward the tissue through free space (e.g., via any suitable optical elements or combination of optical elements, such as a collimating lens, a focusing lens, etc.). As another example, light source 304 can be placed directly onto the tissue.

In some embodiments, system 300 can include multiple modulated light sources 304. For example, system 300 can include additional light sources 304 that each emit light at a different wavelength. In such embodiments, each light source can be coupled to source optical waveguide 308 to be emitted into tissue 324 (and/or can be directed to the same position on tissue 324 as each other light source).

In some embodiments, detector optical waveguide 312 can be any suitable optical fiber, such as one or more single-mode optical fibers. As another example, detector optical waveguide 312 can be implemented using one or more multi-mode fibers. As yet another example, detector optical waveguide 312 can be implemented using one or more few-mode fibers. In some embodiments, detector optical waveguide 312 can be omitted. For example, detector 306 can be directed toward the tissue to receive emitted light through free space (e.g., via any suitable optical elements or combination of optical elements, such as a collimating lens, a focusing lens, etc.). As another example, detector 306 can be placed directly onto the tissue.

Additionally, in some embodiments, source waveguide positioning device 310 and detector waveguide positioning device 314 can be implemented using any suitable device that can be configured to cause light to be emitted by source optical waveguide 308 in a particular direction (e.g., with respect to a surface of a sample 324) and at a particular location (e.g., with respect to sample 324), and can cause detector optical waveguide 312 to receive light from a particular direction (e.g., with respect to a surface of sample 324) and at a particular location (e.g., with respect to sample 324). For example, source waveguide positioning device 310 and detector waveguide positioning device 314 can be implemented using a ferrule, which can be inserted into a housing (not shown) that positions ends of source optical waveguide 308 and detector optical waveguide 312 at particular positions and orientations with respect to sample 324. In some embodiments, source positioning device 310 and/or detector waveguide positioning device 314 can be terminated using an optical element (e.g., a prism, a mirror, etc.) to cause a distal end of the positioning device to substantially contact a surface of the sample (e.g., in cases in which an end of the waveguide is disposed at an angle to the tissue other than perpendicular). In some embodiments, source positioning device 310 and/or detector waveguide positioning device 314 can incorporate any suitable optics that can facilitate sufficient illumination of sample 324 by light emitted from light source 304, such as one or more prisms, one or more mirrors, one or more gradient-index (GRIN) lenses, etc. In some embodiments, photons emitted by source waveguide 308 can be transmitted via a variety of paths 322 through sample 324 that result in photons emitted from source waveguide 308 being received at detector waveguide 314.

In some embodiments, intensity modulated light source 304 can be any suitable light source or combination of light sources that can be configured to emit intensity modulated coherent light suitable for DCS. In some embodiments, light source 304 can emit light at any suitable wavelength, such as near infrared (NIR) wavelengths, infrared (IR) wavelengths, etc. In a more particular example, light source 304 can be implemented using a diode laser that emits light centered around 785 nanometers (nm). As another more particular example, light source 304 can be implemented using a diode laser that emits light at another wavelength in the NIR region, such as 808 nm or 1024 nm. As yet another more particular example, light source 304 can be implemented using a diode laser that emits light at a wavelength in the visible region, such as 660 nm.

In some embodiments, it can be important to operate light source 304 (e.g,. a laser diode) within its linear range throughout the modulation frequencies in order to avoid the influence of harmonics. In some embodiments, this can be accomplished by characterizing the diode frequency characteristics using a Fabry Perot scanning interferometer to determine the linear range of light source 304. However, the sensitivity of the isolator, small misalignments due to presence of an elliptical laser beam (a non-gaussian beam) may introduce errors in the measurement. The Fabry Perot measurements can also confirm that the coherence length of the laser is preserved while modulating the laser at RF frequencies. Accounting for such potential issues can improve the accuracy of FD DCS measurements made using mechanisms described herein. Regardless of whether such potential issues are resolved, FD-DCS is a significant advance of optical technology to retrieve optical properties at single source detector distance in one measurement. Additionally or alternatively, in some embodiments, a linear region of light source 304 can be characterized using other techniques, such as high-resolution sperometry, and/or any other suitable technique that can be use to measure laser line-width, spectrum, and/or coherence length.

In some embodiments, detector(s) 306 can be implemented using any suitable detector or combination of detectors, and can be configured to receive light emitted from sample 324. For example, detector 306 and a proximal end (e.g., with respect to detector 306) of detector waveguide 312 can be positioned such that light that is received from sample 324 by detector waveguide 312 is emitted toward detector 306. As another example, detector 306 can be positioned such that light is received from sample 324 by detector 306 (e.g., via any suitable optical components, such as focusing optics, a narrowband filter, etc.).

In some embodiments, detector 306 can be implemented using any suitable detector capable of detecting the arrival of single photons received at detector 306, and outputting a count of photons received during a particular period of time (e.g., 50 milliseconds (ms), 100 ms, etc.). For example, detector 306 can be implemented using an avalanche photodiode. As another example, detector 306 can be implemented using a single-photon avalanche diode (SPAD). In a more particular example, an Excelitas SPCM AQ4C APD can be used to implement detector 306.

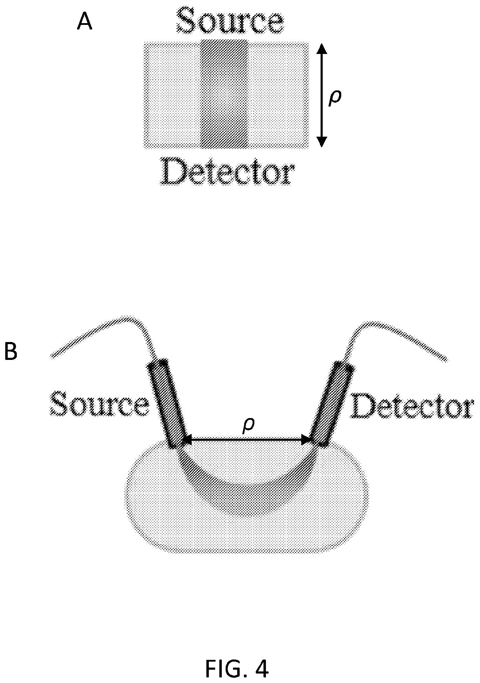

FIG. 4 shows examples of source and detector geometries that can be used in connection with mechanisms described herein for frequency domain diffuse correlation spectroscopy in accordance with some embodiments of the disclosed subject matter.

In some embodiments, FD-DCS can be implemented to measure tissue a transmission geometry (as shown in FIG. 4, panel (A)) or a reflection geometry (as shown in FIG. 4, panel (B). In general, mechanisms described herein are described as using a reflecton geometry, as transmission geometry cannot be used in relatively thick tissues (e. g., $> 8cm$), as the light is fully absorbed in the tissue over long distances distances, and accordingly is impractical for many application. Reflection geometries are more practical for thick tissues, where tissue is assumed to be semi-infinite and homogeneous. In the reflection geometry, a portion of light is reflected back after being absorbed/scattered by multiple cells in the tissue. The reflected light from the tissue can then be collected by an optical waveguide a certain distance away from the source on the tissue surface, which can be referred to as the source detector separation ($\rho$).

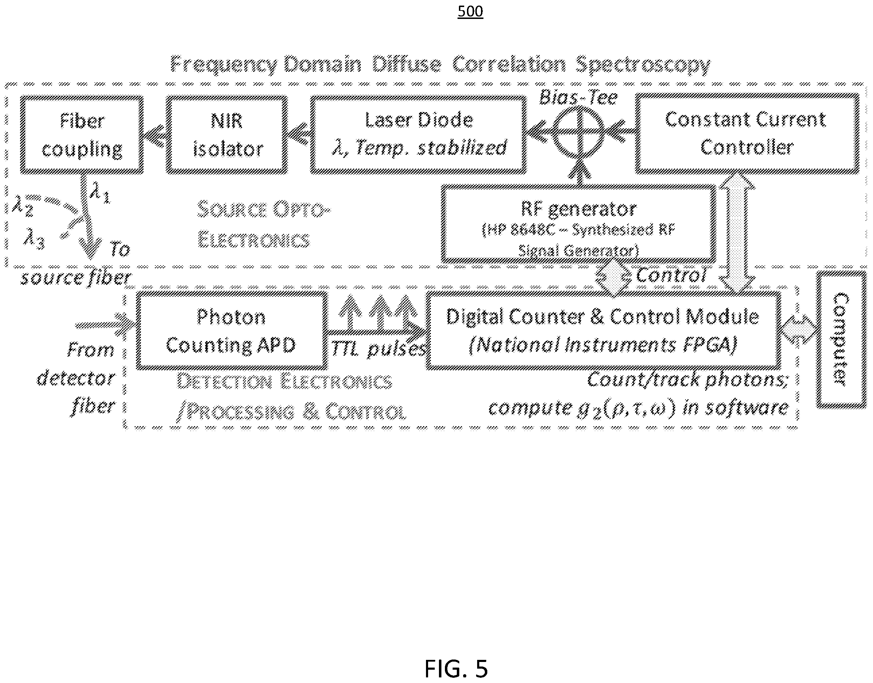

FIG. 5 shows an example 500 of hardware that can be used to implement a system for frequency domain correlation spectroscopy in accordance with some embodiments of the disclosed subject matter.

The hardware shown in FIG. 5 is a particualr example, of a practical implementation of an FD-DCS system implemented in accordance with some embodiments of the disclosed subject matter. For example, system 500 can include a long coherence, near infrared, wavelength stabilized laser (such as a 785 nm laser diode available from Thorlabs headquartered in Netwon, New Jersey, with part number LD785-SEV300, $200 - 300mW$, and coherence length $\approx 10m$) can be used as a light source. Use of the wavelength stabilized laser can ensure that source coherence is sufficient for DCS

measurements. In the impemenation of FIG. 5, the source light is collimated and coupled to a custom fiber-optic probes (implemented using a multi-mode fiber) to illuminate the tissue sample. As shown in FIG. 5, a constant current source (such as a Thorlabs LDC205C current source) was used to drive the laser and a bias-tee was used to add an RF signal generated by a signal generator (such as an HP 8648C from Hewlett Packard, used to implemented a Synthesized RF Signal Generator). In some embodiments, during FD-DCS operation, intensities of the laser source were modulated at 0 hertz (Hz) (e.g., corresponding to continuous wave operation), and modulated at frequencies of 50 MHz to 400 MHz, in steps of 50MHz. Note that this is merely an example, and the laser source can be modulated at more or fewer frequencies and/or with smaller or larger steps between frequencies (and/or at irregular intervals). In some embodiments, the laser source can be modulated at any suitable modulation depth, which can be based on thre linear range of the laser source. For exmaple, operating the laser device in the linear range can ensure that the laser current remains about the threshold current for the device to be in a lasing mode.

As shown in FIG. 5, a free-space polarization sensitive NIR isolator (which can be fiber coupled) can be used to avoid back-reflections into the laser-diode cavity from the fiber coupling. Additionally, as shown in FIG. 5, a temperature controller (such as a Thorlabs TED200C) was used to maintain the laser diode temperature at room temperature. In some embodiments, the isolator and temperature controller together can help the laser diode maintain single frequency operation and thereby its coherence length. As shown in FIG. 5, in some embodiments, modulated lasers sources with multiple other wavelengths (e.g., $\lambda_2$ and $\lambda_3$) can also be emitted toward the sample (e.g., via the source fiber and any suitable coupling optics, such as one or more fiber couplers, an optical switch, electronically controlling the laser sources to operate during different time periods, etc.). In some embodimetns, measurments of received intensity can be recorded for each wavelength, and at each of various modulation frequencies. For example, a laser source for each wavelength can be included in system 500. In some embodiments, measurements of tissue properties (e.g., $\mu_a$ and/or $\mu_s$) at different wavelengths can be used to estimate properties, such as oxy-hemoglobin and deoxy-hemoglobin.

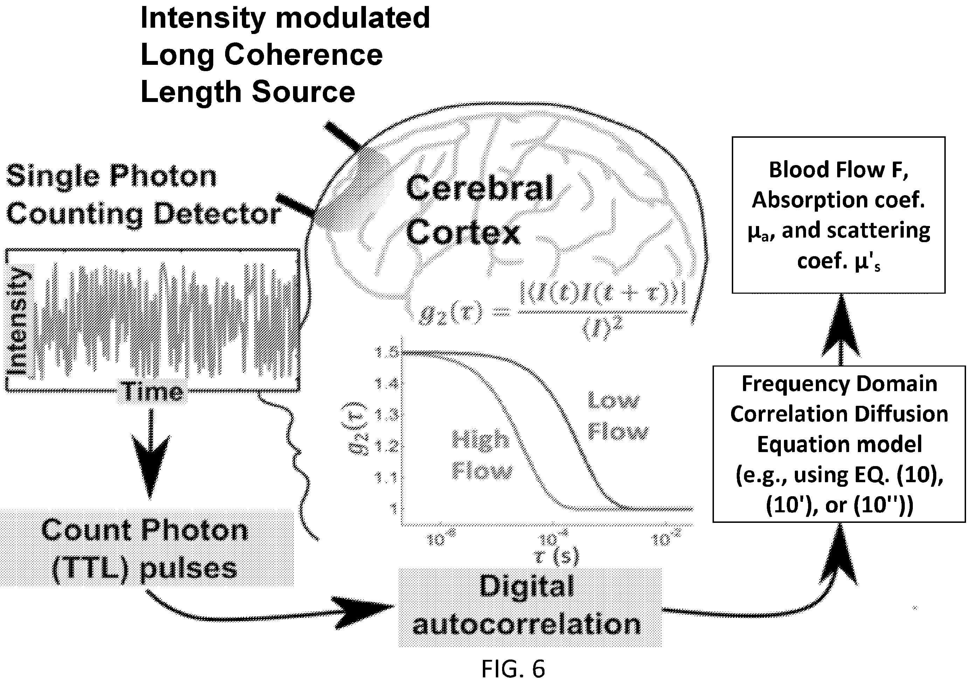

In some embodiments, back-scattered light can be collected at a detector position a particular distance from the position at which the source light is emitted. For example, in the example of FIG. 5, the detector fiber is positioned 1.3 cm away from the source on the sample using single-mode detector fibers and directed to single photon counting avalanche photo diode (APD) (such as an Excelitas SPCM AQ4C APD), which are often used in conventional DCS instruments. The APDs generate TTL pulses for every photon detected, which can be counted using a counter/timer module (such as a National Instruments PXI-6229 counter/timer). In some embodiments, software (such as LabVIEW) can be used to acquire digital pulses at every modulation frequency using a digital counter/timer module (such as a digital counter/timer module from National Instruments) with up to 20$Hz$ acquisition rates, sampled at 1 MHz (e.g., couting a number of photons received over each 1 microsecond ($\mu s$) period for each modulation frequency for 50 ms, each, which can result in tens of thousands of count detection values for each modulation frequency) to compute a DCS intensity auto-correlation function (e.g., as described below in connection with FIG. 6). In some embodiments, system 500 can determine $\mu_a$, $\mu_s'$, and $F$ based on a fit between the intensity auto-correlation function to a Frequency Domain Correlation Diffusion Equation model described below in connection with FIG. 6. In some embodiments, system 500 can collect a predetermined number of frames of data (e.g., 50 ms of count values, each corresponding to a smaller period of time, such as 1 $\mu s$). For example, system 500 can collect 1 frame of data, or up to 10 frames of data, for each modulation frequency (e.g., for each wavength laser source).

FIG. 6 shows an example of frequency domain diffuse correlation spectroscopy in accordance with some embodiments of the disclosed subject matter. In the particular example of FIG. 6, mechanisms described herein are used to measure properties of brain tissue. However, this is merely an example, and mechanisms described herein can be used to measure properties of any suitable tissue.

In general, DCS is a complementary optical technique to measure tissue dynamics, particularly blood flow ($F$). DCS can measure temporal intensity fluctuations of highly coherent laser light scattered through tissue. Light diffuses through the tissue via multiple path-lengths from the source to the detector. At the detector, electric fields of the multiply scattered light via multiple path-lengths interfere causing a "speckle" pattern to develop at the detector. When the scattering agents are dynamic (e.g., red blood cells), the speckle pattern, and the intensities, fluctuate in time. To quantify blood flow, the auto-correlation functions of these fluctuating intensities can be calculated, and fit to a diffusion model. Fast decay of the intensity auto-correlation function implies high flow while slower decay implies low flow. The normalized intensity auto-correlation function that is measured at the detector can be expressed as:

$$g_2(\tau) = \frac{\langle I(t)I(t+\tau)\rangle_t}{\langle I(t)\rangle^2} \qquad (4)$$

In the example of DCS, $I(t)$ is intensity at the detector at time t (e.g., measured based on the count of photons received over a particular period of time at a particular sampling rate) and $I(t+\tau)$ is time shifted intensity with a delay time $\tau$. As described above, conventional DCS can be used to estimate flow $F$, and cannot be used to measure absorption or scattering properties of the tissue.

The diffusive process in DCS can be modeled by a correlation diffusion equation (CDE) which can be expressed as:

$$[\nabla^2 D - v\mu_a - 2v\mu_{s'}\kappa_o^2 F\tau]G_1(\tau) = -vS_o. \tag{5}$$

Here, $G_1(\tau)$ is the electric field auto-correlation function, $\mu_a$ and $\mu'_s$ are the tissue absorption and scattering coefficients, $D$ is photon diffusion coefficient, $v$ is the speed of light in tissue, $\kappa_o = 2\pi/\lambda$ is the wave number of input light, $\tau$ is the correlation lag, $S_o$ is the source power, and $F$ is the tissue blood flow index.

The solution to the CDE is dependent on the source type and the geometry. The intensity auto-correlation function is related to the electric field auto-correlation function using Siegert's relation, and can be expressed as:

$$g_2(\tau) = 1 + \beta |g_1(\tau)|^2, \tag{6}$$

where $g_1(\tau)$ is the normalized electric field auto-correlation function, which can be expressed as:

$$g_1(\tau) = \frac{\langle E(t)E(t+\tau)\rangle_t}{\langle E(t)\rangle^2} \tag{7}$$

In general, intensity $I$ can be related to the electric field $E$ by $I = |E|^2$.

In some embodiments, estimating both blood flow and oxygenation using mechanisms described herein can be facilitated using a generalized mathematical model that can characterize the diffusion of coherent intensity fluctuations through tissue. As a starting point, the steady-state (time independent) correlation diffusion equation $[\nabla^2 D - v\mu_a - 2v\mu'_s k_0^2 F\tau]G_1(\rho, \tau) = -vS_0$ described above in EQ. (5) can be considered, which describes and establishes that the auto-correlation of the electric field ($G_1(\rho, \tau)$) diffuse through tissue much like the fluence rate in the photon diffusion equation.

Here, $G_1(\tau)$ is the electric field auto-correlation function, $\mu_a$ and $\mu'_s$ are the tissue absorption and reduced scattering coefficients respectively, $D$ is photon diffusion coefficient, $v$ is the speed of light in tissue, $\kappa_o = 2\pi/\lambda$ is the wave number of input light, $\tau$ is the correlation lag, $S_o$ is the source power and $F$ is the tissue blood flow index.

The Photon Diffusion Equation (PDE) for a continuous wave source, which can be expressed by $[\nabla^2 D - v\mu_a]\phi_{cw} = -vS_o$, where $\phi_{cw}$ is photon fluence rate for continuous wave source, and other terms are as defined above. The Correlation Diffusion Equation (CDE) can be characterized as an extension of the continuous wave PDE, but with the notable addition of a dynamic absorption term $2v\mu_{s'}\kappa_o^2 F\tau$. Further, the PDE for an intensity modulated source can be extended from the continuous wave PDE by addition of an $i\omega$ term, which can be expressed as: $[\nabla^2 D - v\mu_a - i\omega]\phi_{ac} = -vS_{ac}$. $\omega\pi f$ is the frequency of modulation in Hz and $S_{ac} = S_o(1 + \text{m.} \ e^{i\omega t})$, m is the modulation depth of the source ($0 \le m \le 1$).

Additionally, adding $i\omega$ to the CDE, an expression for the Frequency domain Correlation Diffusion Equation (FD-CDE) can be derived, which extends DCS to intensity modulated sources, and can be expressed as:

$$[\nabla^2 D - v\mu_a - 2v\mu_{s'}\kappa_o^2 F\tau - i\omega]G_1(\tau) = -vS_{ac}. \tag{8}$$

The modulated intensity travels through the tissue as a wave and can be called Diffuse Correlation Density Wave (DCDW), similar to Diffuse Photon Density Wave (DPDW). The solution to the DCDW for semi-infinite homogeneous tissue can be expressed as:

$$G_1(\rho, \tau, \omega) = \frac{v}{4\pi D}\left[\frac{exp(-K_D(\tau,\omega)r_1)}{r_1} - \frac{exp(-K_D(\tau,\omega)r_b)}{r_b}\right], \tag{9}$$

were, $\rho$ is source-detector separation, $K_D(\tau, \omega) = [(v\mu_a - 2v\mu_{s'}\kappa_o^2 F\tau - i\omega)/D]^{1/2}$ is frequency dependent dynamic wave vector, $r_1 = (l_{tr} + \rho^2)^{\frac{1}{2}}$; $r_b = ((2z_b + l_{tr})^2 + \rho^2)^{\frac{1}{2}}$, $l_{tr} \approx 1/\mu'_s$ is transport mean free path, and $Z_b = \frac{2}{3}l_{tr}\frac{1+R_{eff}}{1-R_{eff}}$ is extrapolated zero boundary for index matched boundaries.

In some embodiments, the frequency dependent normalized intensity auto-correlation function is calculated by substituting for normalized version of $G_1(\rho, \tau, \omega)$ in the Siegert relation, and the intensity auto-correlation function can be calculated using the following expression:

$$g_2(\rho, \tau, \omega) = 1 + \beta \left| \frac{G_1(\rho, \tau, \omega)}{G_1(\rho, 0, 0)} \right|^2, \tag{10}$$

where $\beta$ is an instrumentation factor that depends on speckle averaging. This expression of $g_2(\rho, \tau, \omega)$ can be characterized as a general expression for the frequency dependent intensity auto-correlation function. Since $g_2(\rho, \tau, \omega)$ is normalized to the average intensity, FD-DCS can be less sensitive to external factors like fiber-tissue coupling coefficients.

This frequency dependent solution for $g_2(\rho, \tau, \omega)$ can arguably be characterized as the most general equation describing diffusion of coherent light through tissue. In the limiting case of $\tau = 0$ or $F = 0$, the DCDW can reduce to the DPDW. Additionally, the solution $g_2(0, \omega)$ is analogous to a frequency dependent solution to the DPDW. Conventional implementations of DCS are also a limiting case ($\omega = 0$), as such implementations utilize a solution to a "DC" or Continuous Wave (CW) Correlation Diffusion Equation. Additionally, note that instrumentation required to implement FD-DCS is considerably simpler than the instrumentation used to implement Time Domain Diffuse Correlation Spectroscopy (TD-DCS). For example, FD-DCS does not require a specialized highly coherent pulsed laser, and its measurements can be performed with conventional DCS detectors.

Note that in frequency domain-based implementation of diffuse optical spectroscopy (FD-DOS), the AC component of a signal can be relatively easily filtered from the DC component (e.g., using a DC blocking capacitor). Accordingly, solutions for FD-DOS typically only include expressions for the measured "ac" fluence. However, this is not possible in DCS because conventional DCS instruments record photon counts using a single photon counting avalanche photo diode, and thus the recorded signal contains both AC and DC components, which can be accounted for via the modulation depth (m).

In some embodiments, the frequency dependent normalized intensity auto-correlation function can also account for the modulation depth by modeling the measured intensity auto-correlation function, which can be expressed as:

$$g_2(\tau, \rho, \omega) = 1 + \beta[(1 - m)|g_1(\rho, \tau, \omega = 0)|^2 + (m)|g_1(\rho, \tau, \omega = 0)|^2] \tag{10'}$$

While the empirical model in EQ. (10') can account for the influence of the modulation depth, this model works to a first approximation. A more robust expression can potentially improve the accuracy of an FD-DCS system implemented in accordance with some embodiments of the dislcosed subject matter.

In some embodiments, a rigorous expression for the intensity auto-correlation function that is derived directly from the electric fields can be used in an FD-DCS system. Recall that $G_1(\tau, \omega)$ is the un-normalized electric field auto-correlation function, and $G_2(\tau, \omega)$ is the un-normalized intensity auto-correlation function. Note that, in practice, only the latter is measured.

The electric field reaching the detector can be characterized as a superposition of electric field due to continuous wave and modulating source. Therefore,

$$E(t) = E_{dc}(t) + E_{ac}(t) \tag{11}$$

The temporal intensity auto-correlation function can be written as

$$G_2(\tau) = \langle I(t)I(t + \tau)\rangle_t \tag{12}$$

Inserting EQ. (11) in EQ. (12) ($I = E^2$), and expanding yields 16 terms.

$$
\begin{aligned}
G_{2FD}(\tau) &= \langle I(t)I(t + \tau)\rangle_t \\
&= \langle E(t)E^*(t)E(t + \tau)E^*(t + \tau)\rangle_t \\
&= \langle (E_{dc}(t) + E_{ac}(t))(E_{dc}^*(t) + E_{ac}^*(t)) \\
&\quad (E_{dc}(t + \tau) + E_{ac}(t + \tau))(E_{dc}^*(t + \tau) + E_{ac}^*(t + \tau))\rangle_t \\
&= \langle (E_{dc}(t)E_{dc}^*(t) + E_{dc}(t)E_{ac}^*(t) + E_{ac}(t))E_{dc}^*(t) + E_{ac}(t))E_{ac}^*(t)) \\
&\quad (E_{dc}(t + \tau)E_{dc}^*(t + \tau) + E_{dc}(t + \tau)E_{ac}^*(t + \tau) \\
&\quad +E_{ac}(t + \tau))E_{dc}^*(t + \tau) + E_{ac}(t + \tau))E_{ac}^*(t + \tau)))\rangle_t
\end{aligned} \tag{13}
$$

$$\begin{aligned}
G_{2FD}(\tau) = \langle & E_{dc}(t)E_{dc}^*(t)E_{dc}(t+\tau)E_{dc}^*(t+\tau) + E_{dc}(t)E_{dc}^*(t)E_{dc}(t+\tau)E_{ac}^*(t+\tau) \\
& +E_{dc}(t)E_{dc}^*(t)E_{ac}(t+\tau)E_{dc}^*(t+\tau) + E_{dc}(t)E_{dc}^*(t)E_{ac}(t+\tau)E_{ac}^*(t+\tau) \\
& +E_{ac}(t)E_{dc}^*(t)E_{dc}(t+\tau)E_{dc}^*(t+\tau) + E_{ac}(t)E_{dc}^*(t)E_{dc}(t+\tau)E_{ac}^*(t+\tau) \\
& +E_{ac}(t)E_{dc}^*(t)E_{ac}(t+\tau)E_{dc}^*(t+\tau) + E_{ac}(t)E_{dc}^*(t)E_{ac}(t+\tau)E_{ac}^*(t+\tau) \\
& +E_{dc}(t)E_{ac}^*(t)E_{dc}(t+\tau)E_{dc}^*(t+\tau) + E_{dc}(t)E_{ac}^*(t)E_{dc}(t+\tau)E_{ac}^*(t+\tau) \\
& +E_{dc}(t)E_{ac}^*(t)E_{ac}(t+\tau)E_{dc}^*(t+\tau) + E_{dc}(t)E_{ac}^*(t)E_{ac}(t+\tau)E_{ac}^*(t+\tau) \\
& +E_{ac}(t)E_{ac}^*(t)E_{dc}(t+\tau)E_{dc}^*(t+\tau) + E_{ac}(t)E_{ac}^*(t)E_{dc}(t+\tau)E_{ac}^*(t+\tau) \\
& +E_{ac}(t)E_{ac}^*(t)E_{ac}(t+\tau)E_{dc}^*(t+\tau) + E_{ac}(t)E_{ac}^*(t)E_{ac}(t+\tau)E_{ac}^*(t+\tau) \rangle_t
\end{aligned} \tag{14}$$

Note that 10 of these products (terms 2,3,4,5,8,9,12,13,14, and 15) average to zero. Thus $G_{2FD}(\tau)$ can be simplified to

$$\begin{aligned}
G_{2FD}(\tau) = \langle & E_{dc}(t)E_{dc}^*(t)E_{dc}(t+\tau)E_{dc}^*(t+\tau) \\
& +E_{ac}(t)E_{dc}^*(t)E_{dc}(t+\tau)E_{ac}^*(t+\tau) \\
& +E_{ac}(t)E_{dc}^*(t)E_{ac}(t+\tau)E_{dc}^*(t+\tau) \\
& +E_{dc}(t)E_{ac}^*(t)E_{dc}(t+\tau)E_{dc}^*(t+\tau) \\
& +E_{dc}(t)E_{ac}^*(t)E_{ac}(t+\tau)E_{ac}^*(t+\tau) \\
& +E_{ac}(t)E_{ac}^*(t)E_{ac}(t+\tau)E_{ac}^*(t+\tau) \rangle_t
\end{aligned} \tag{15}$$

Here, terms 1 and 6 in EQ. (15) are readily seen as representing the auto-correlation of DC and AC terms alone, while and terms 3 and 4 are cross terms. The terms 2 and 4 can convert to intensity terms as shown below

$$G_{2FD}(\tau) \approx \langle I_{dc}^2 \rangle_t + \beta \langle I_{dc}^2 \rangle_t |g_{1dc}(\tau)|^2 + 2I_{dc}I_{ac} + 2\beta I_{dc}I_{ac}|g_{1dc}||g_{1ac}| \\
+ \langle I_{ac}^2 \rangle_t + \beta \langle I_{ac}^2 \rangle_t |g_{1ac}(\tau)|^2 \tag{16}$$

In FD-DCS the source is a modulating source and given by $S_{ac} = S_o(1 + m.e^{i\omega t})$, implying $I = I_{ac} + I_{dc}$ and $I_{ac}/I_{dc} = m$. The normalized intensity auto-correlation function after substituting m in EQ. (16) is

$$g_{2_{FD}}(\rho, \tau, \omega) = 1 + \beta \left[ \frac{|g_{1dc}(\tau)|^2 + 2m|g_{1dc}||g_{1ac}| + m^2|g_{1ac}(\tau)|^2}{(1+m)^2} \right] \tag{10''}$$

Note here, that for a conventional CW-DCS, $g_{1ac} = 0$ and $m = 0$, in which case $g_{2FD}(\rho, \tau, \omega = 0)$ reduces to $g_2(\rho, \tau) = 1 + \beta|g_{1dc}(\tau)|^2$, which is the well known Siegert relation.

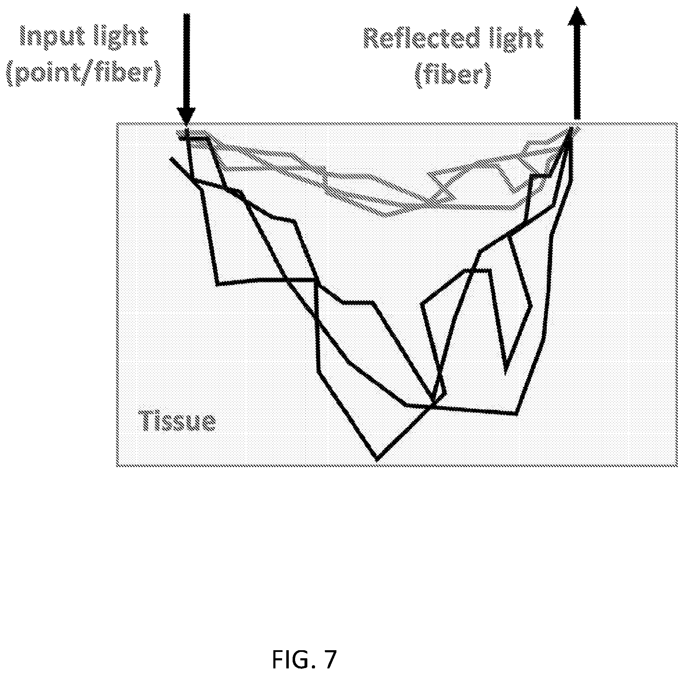

FIG. 7 shows an example of different path lengths that photons emitted substantially simultaneously can take through a tissue sample which can result in phase differences between detected photons in a system for frequency domain diffuse correlation spectroscopy in accordance with some embodiments of the disclosed subject matter.

As shown in FIG. 7, photons that are emitted from the source (e.g., via source waveguide 308) substantially simultaneously can take different paths to a detector (e.g., detector 306 via detector waveguide 312. Additionally subsequent photons that are emitted can take slightly different paths, which may be due to differences in the sample, differences in the initial angle at which the photon is emitted, etc. Accordingly, photons that arrive at the detector simultaneously are likely to arrive with different phases due to the differences in path length can cause the received photons, which initially had the same phase when emitted. As the modulation frequency is changed, the relationship between phases of photons received at the detector can change.

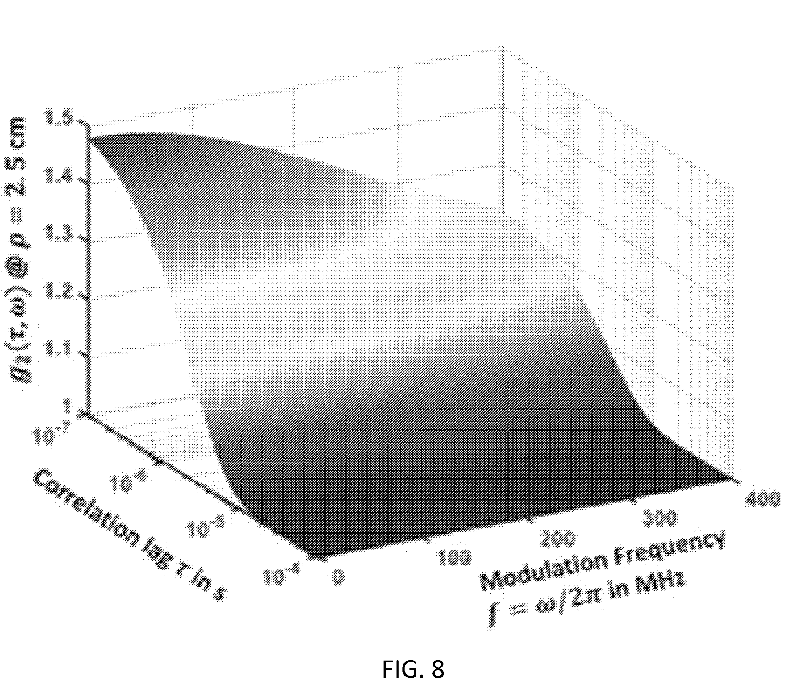

FIG. 8 shows an example of numerically calculated frequency dependent intensity auto-correlation function for various modulation frequencies and time lags in accordance with some embodiments of the disclosed subject matter.

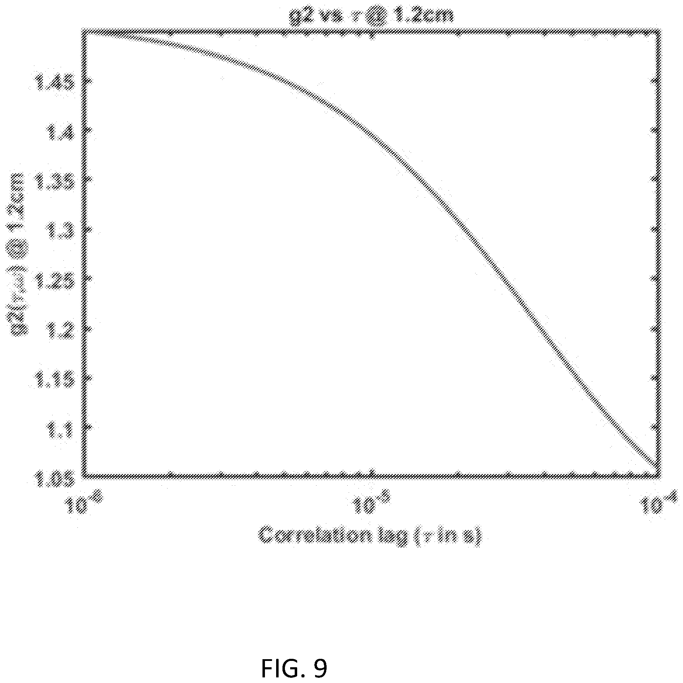

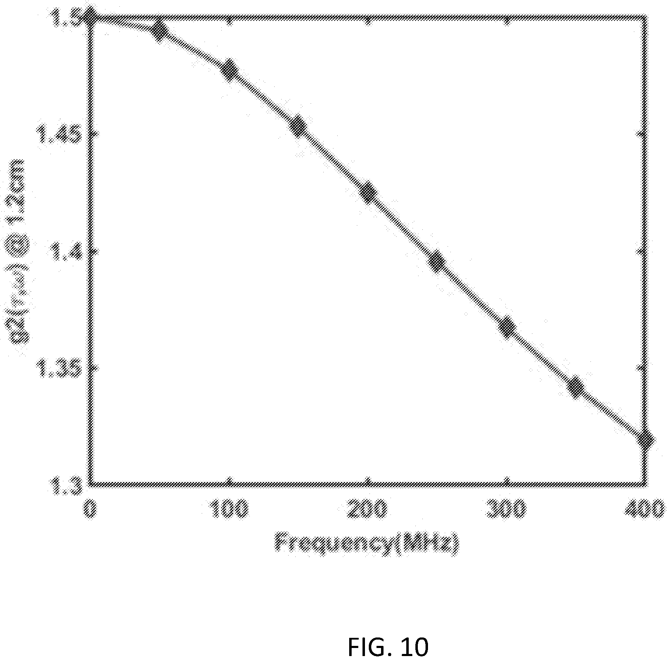

Numerical computation of the frequency dependent intensity auto-correlation function $g_2(\rho, \tau, \omega)$ (e.g., as described above in connection with EQ. (10)) at $\rho = 2.5$ cm for a flow index of $F = 10^{-7}$ cm²/sec, $\beta = 0.5$, $\mu_a = 0.1$ cm⁻¹ and $\mu_{s'} = 10$ cm⁻¹ is shown in FIG. 8. This can be better understood by looking at FIGS. 9 and 10. For example, FIG. 9 gives the traditional $g_2(\rho, \tau)$ with $\omega = 0$ and FIG. 10 is the simulated $g_{2FD}(\rho, \tau, \omega)$ intercept as a function of $\omega$.

FIG. 9 shows an example of a numerically calculated frequency dependent intensity auto-correlation function at zero frequency as a function of time lag in accordance with some embodiments of the disclosed subject matter.

FIG. 10 shows an example of a numerically calculated intercept of the frequency dependent intensity auto-correlation function as a function of modulation frequency in accordance with some embodiments of the disclosed subject matter.

FIG. 11 shows an example 1100 of a process for frequency domain diffuse correlation spectroscopy in accordance with some embodiments of the disclosed subject matter.

As shown in FIG. 11, process 1100 can start at 1102 by emitting relatively long-coherence laser light that is modulated at multiple frequencies toward a sample. For example, process 1100 can cause the light source to be modulated at various frequencies (e.g., frequencies in the RF range from 0-600 MHz). In such an example, process 1100 can use a frequency generator to modulate the laser light source, as described above in connection with FIGS. 3 and 5. As described above in connection with FIGS. 3-6, process 1100 can utilize a waveguide to emit light toward the sample at a particular location.

At 1104, process 1100 can include collecting light using one or more detectors located a distance $\rho$ from a location at which the light is emitted. In some embodiments, process 1100 can detect, for each modulation frequency, photons arriving at the detector during a period at which the modulation frequency is being applied to the light source.

As described above in connection with FIGS. 3, 5, and 6, process 1100 can receive counts of photons detected by a single photon detector (e.g., an APD, a SPAD), which can be indicative of intensity changes over time.

As described above, in some embodiments, at 1104, process 1100 can collect photon counts at a particular sampling rate (e.g., 500 kilohertz (kHz), 1 MHz, 2 MHz, etc.) for a particular period of time during which light emitted at a particular intensity modulation frequency (e.g., 50 ms, 100 ms, etc.).

At 1106, process 1100 can determine an intensity auto-correlation function for each modulation frequency. In some embodiments, the intensity auto-correlation function can be calculated using any suitable intensity auto-correlation function, such as the normalized intensity auto-correlation function described above in connection with EQ. (4).

At 1108, process 1100 can estimate various tissue properties, such as flow F, absorption coefficient $\mu_a$, and scattering coefficient $\mu_s^t$ using the intensity auto-correlation functions calculated at 1106, using any suitable technique or combination of techniques. For example, process 1100 can model a frequency dependent normalized intensity auto-correlation function (e.g., as described above in connection with one of EQS. (10), (10'), or (10")). In such an example, numerical techniques can be used to fit the various values of the intensity auto-correlation functions to the frequency dependent normalized intensity auto-correlation function by optimizing for values of F, $\mu_a$, and $\mu_s^t$ that produce the calculated intensity auto-correlation functions.

At 1110, process 1100 can cause the estimated tissue parameters to be presented using any suitable technique or combination of techniques. For example, process 1100 can cause the tissue parameters to be presented using a display (e.g., a display of a device executing process 1100, a display of a device coupled to a device executing process 1100, etc.). Additionally or alternatively, in some embodiments, one or more parameters (e.g., oxy-hemoglobin $C_{HbO}$, $C_{HbR}$, $StO_2$, tissue metabolism rate of oxygen, etc.) derived from the estimated tissue parameters (e.g., $\mu_a$, $\mu_s^t$, F).

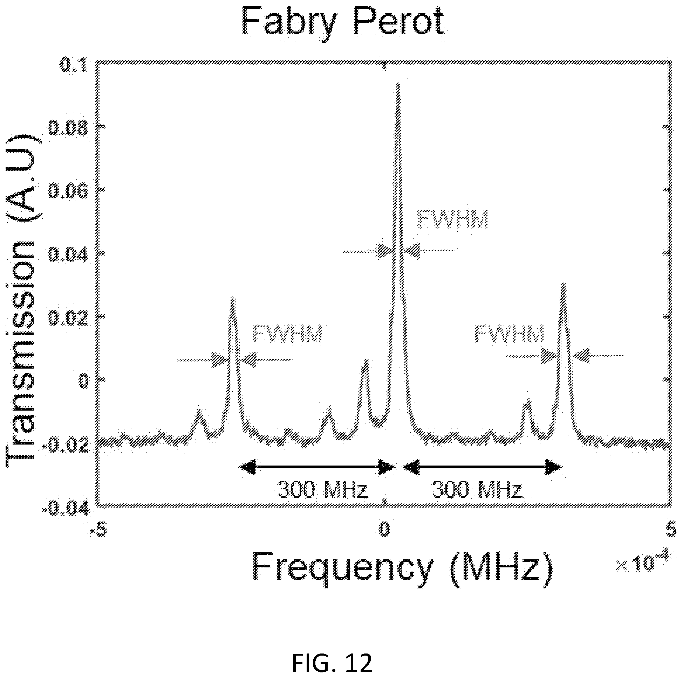

FIG. 12 shows an example Fabry-Perot plot of an intensity modulated laser source modulated at 300 megahertz.

Note that FD-DCS introduces a significant change in the instrumentation of a DCS blood flow monitoring system, as the intensity of the laser source is modulated at RF frequencies. A first experiment confirmed that the light source maintained its coherence length at these modulation frequencies. The presence of an optical isolator after the laser the diode helps reduce back reflections from fibers/sample to the diode laser cavity. This may be essential to maintaining single-frequency operation of the laser diode even when the laser intensity is not modulated. To verify that the single-frequency operation is maintained during intensity modulation, the line-width of the laser using a Fabry-Perot interferometer was characterized. A Fabry-Perot interferometer, also known as multiple beam interferometer, detects the laser spectrum with high precision using interference from multiple beams generated from two highly reflective glass with partial transmission facing each other.

A scanning Fabry-Perot interferometer (such as a Thorlabs SA30-73,) was used to characterize the laser source. This instrument has a wavelength range of 630 - 824 *nm,* a free spectral range of 1.5 *GHz,* and resolution of less than 1 *MHz.* It therefore has sufficient range to observe the side lobes produced by modulating frequencies up to 700 MHz. The optical beam from the laser was collimated and steered into the interferometeric cavity. A controller (a Thorlabs SA201) was used to adjust the length of the cavity by adjusting supply voltage to the piezoelectric transducers present on the interferometer. The controller also generates an alternating voltage (triangle or saw-tooth) to scan the length of the cavity accounting for one spectral range of the interferometer (Free spectral range of FSR). The transmitted light intensity was measured using a photo diode followed by a trans-impedance amplifier. A trigger/control signal from the controller was used to isolate one FSR for display on an oscilloscope for post processing.

FIG. 12 shows the spectrum of the laser from a fabry-perot interferometer experiment with the laser source modulated at 300 *MHz.* The y-axis is the transmission intensity in arbitrary units. The x-axis represents one trigger period, equal to the free spectral range. A prominent central lobe and two side lobes are present clearly visible. The central peak represents the "dc" operation of the laser, while the two side lobes represent the "ac" components. Single-frequency operation can be confirmed by measuring the line width of the central and side lobes. TABLE 1 shows the line-width of the laser for all three lobes, measured as the full-width at half-max (FWHM) for different modulating frequencies. The line-width of the laser stays relatively constant with modulating frequency, indicating that intensity modulating the laser source does not impact the coherence length.

TABLE 1: Line-width of intensity modulated laser source at multiple frequencies.

| Frequency (MHz) | Left Sidelobe (MHz) | Center Peak (MHz) | Right Sidelobe (MHz) |
|---|---|---|---|
| DC | - | 100 | - |
| 50 MHz | 110 | 230 | 110 |
| 100 MHz | 100 | 400 | 100 |
| 150 MHz | 100 | 170 | 100 |
| 200 MHz | 110 | 320 | 125 |
| 250 MHz | 160 | 110 | 140 |
| 300 MHz | 125 | 100 | 160 |
| 350 MHz | 90.9 | 166 | 125 |
| 400 MHz | 110 | 160 | 111 |

FIG. 13 shows an example of measured frequency dependent intensity auto-correlation function using a system for frequency domain diffuse correlation spectroscopy implemented in accordance with some embodiments of the disclosed subject matter.

The FD-DCS instrument and model was also validated using tissue simulating phantoms to verify the accuracy of the device to estimate static and dynamic tissue optical properties (tissue absorption coefficient $\mu_a$, tissue scattering coefficient $\mu_s'$, and blood flow index $F$). A liquid phantom, comprising India ink, 20% intralipid, and distilled water was used for this validation.

Frequency dependent DCS intensity auto-correlation functions were recorded at each wavelength $g_2^m(\tau, \rho, \omega)$. Superscript $m$ indicates measured data. The measured data was fit to the model describing frequency dependent intensity auto-correlation function $g_2^{th}(\tau, \rho, \omega)$, superscript $th$ indicates theory, to estimate free parameters $\mu_a(\lambda_1)$, $\mu_s(\lambda_1)$ and F. The fit was solved as a multivariate nonlinear optimization problem, wherein parameter $\chi^2 = \Sigma_{MxN}|g_2^{th}(\tau, \rho, \omega) - g_2^m(\tau, \rho, \omega)|^2$ was minimized over N correlation lags, and M modulation frequencies to estimate unknown parameters (fminsearch, MATLAB, Natick, MA). Additional fit parameters include $\beta$ the speckle averaging factor and m the modulation depth. When translated to in-vivo experiments, concentrations $C_{HbO}$, $C_{HbR}$ can be estimated from measured $\mu_a(\lambda)$ and molar extinction coefficients $\varepsilon(\lambda)$. To elaborate, the linear system of equations given by $\mu_a(\lambda) = \varepsilon_{HBR}(\lambda)C_{HbR} + \varepsilon_{HbO}(\lambda)C_{HbO}$ where $C_{HbO}$, $C_{HbR}$ corresponds to oxy- and deoxy- hemoglobin. Finally, tissue oxygen saturation is $StO_2 = C_{HbO}/(C_{HbO} + C_{HbR})$, and blood flow.

A prototype FD-DCS instrument described above in connection with FIG. 5 was used to record $g_2(\tau, \rho, \omega)$ from the phantom with a custom fiber optic probe, with a source detector separation ($\rho = 1.2cm$), placed on the surface of the liquid (semi-infinite geometry). The integration time of the measurements was 100 ms, while the average intensity across the modulation frequencies was $300KHz \pm 100KHz$. 180 intensity auto-correlation curves were averaged (per frequency) to improve measurement signal to noise ratio. FIG. 12 shows frequency dependent DCS intensity auto correlation functions measured from a representative liquid phantom with optical properties $\mu_a$ = 0.12$cm^{-1}$ and $\mu_s' = 10cm^{-1}$ at $785\,nm$. The measured auto-correlation function data points are represented by circles, and each color/curve represents measurements made with different modulation frequencies from 0 $Hz$ to 400 $MHz$ in steps of 50$MHz$. The x-axis represents correlation lag (1 $\mu s$ to 100 $\mu s$) displayed on a log scale. The solid lines are fits of the data to the frequency dependent solution to the correlation diffusion equation (FD-CDE). The nonlinear fitting estimated the phantom optical properties to be $\mu_a$ = 0.12$cm^{-1}$ and $\mu_s' = 10cm^{-1}$. The optical properties were recovered within 5.3% error for $\mu_a$ and 9% for $\mu_s'$. The estimated blood flow index of $F$ = 2.88 x 10$^{-8}cm^2/s$ is in the rage of flow indices typically observed in these liquid phantoms.

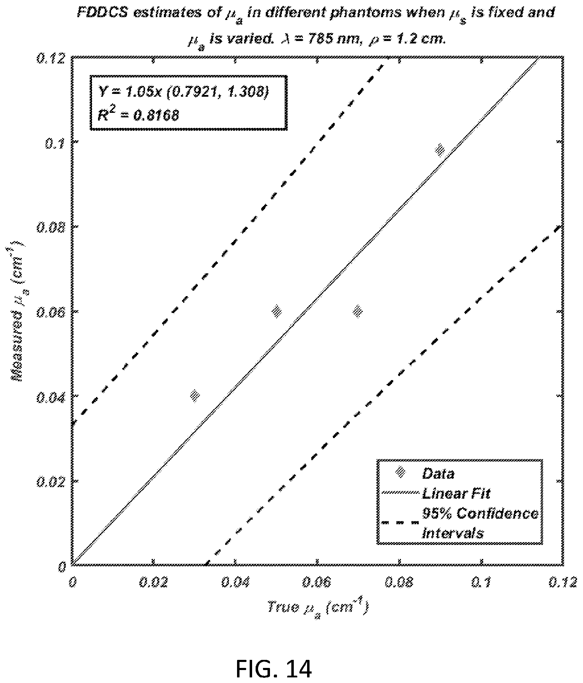

FIG. 14 shows an example plot of sensitivity to absorption changes in tissue simulating phantoms when absorption coefficient is varied and scattering coefficient is kept constant of a frequency domain diffuse correlation spectroscopy system implemented in accordance with some embodiments of the disclosed subject matter.

A second tissue phantom experiment was performed in which the linearity and sensitivity of FD DCS was recorded/estimated to estimate changes in tissue optical properties. In a series of experimental sessions (4 steps each), the static and dynamic properties of the phantoms were systematically varied, while estimating $\mu_a$, $\mu_s'$, and $F$ from the measurements of $g_2(\tau, \rho, \omega)$ from EQ. (10'). Specifically, $\mu_a$ was varied from 0.03 to 0.9$cm^{-1}$ in steps of 0.02$cm^{-1}$ by changing the concentration of India ink in the phantom. Additionally, $\mu_s'$ was varied from 60 to 140$cm^{-1}$ in steps of 20$cm^{-1}$ by changing the volume of intralipid in the phantom $F$ was held constant across all phantoms. As described above, 9 frequency dependent intensity auto-correlation functions were measured and averaged per frequency for all phantoms. Non-linear fitting of the measured data to EQ. (10') yielded the estimates for $\mu_a$, $\mu_s'$, and $F$. In all cases, the optical properties recovered using the FD-DCS instrument were compared to theoretical values. The relationship between measured and set tissue properties of the phantom will indicate the sensitivity of FD-DCS instrument to changes in $\mu_a$ and $\mu_s'$.

FIG. 14 plots the phantom absorption coefficients ($\mu_a$) estimated with FD-DCS on the y-axis, with the theoretical values along the x-axis. $\mu_s'$ and $F$ were fixed for these phantoms. The solid diamond red markers indicate the estimated absorption coefficient, while the blue line is a linear fit to the data. The dashed black lines indicate the 95% confidence bounds of the linear regression. The slope of the regression line is 1.05 ($R^2 > 0.8$) indicating good 1:1 correspondence between measured and actual values of absorption coefficients. The results show that FD-DCS estimates of $\mu_a$ are linear.

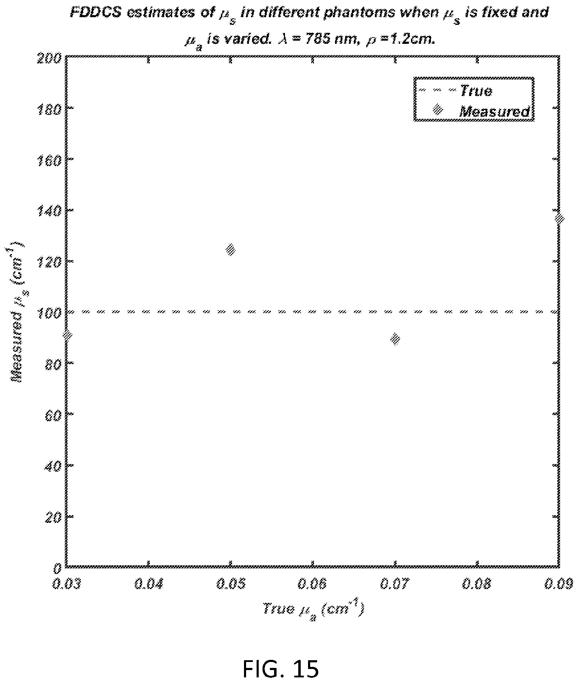

FIG. 15 shows an example plot of sensitivity to scattering changes in tissue simulating phantoms when absorption coefficient is varied and scattering coefficient is kept constant of a frequency domain diffuse correlation spectroscopy system implemented in accordance with some embodiments of the disclosed subject matter.

FIG. 15 shows the estimates of $\mu_s$ by FD-DCS system for different phantoms when $\mu_a$ is varied and $\mu_s$ is fixed. As expected the phantom scattering coefficients are close to their true values.

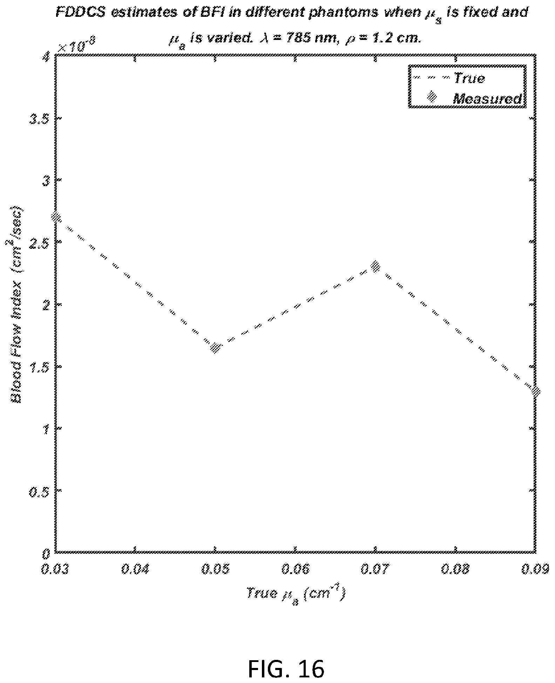

FIG. 16 shows an example plot of sensitivity to BFI changes in tissue simulating phantoms when absorption coefficient is varied and scattering coefficient is kept constant of a frequency domain diffuse correlation spectroscopy system implemented in accordance with some embodiments of the disclosed subject matter.

FIG. 16 shows the estimates of BFI by FD-DCS system for different phantoms when $\mu_a$ is varied and $\mu_s$ is fixed. The blood flow index are in the order of 1e-8 as expected and scattering coefficient for various phantoms in these phantoms were relatively constant.

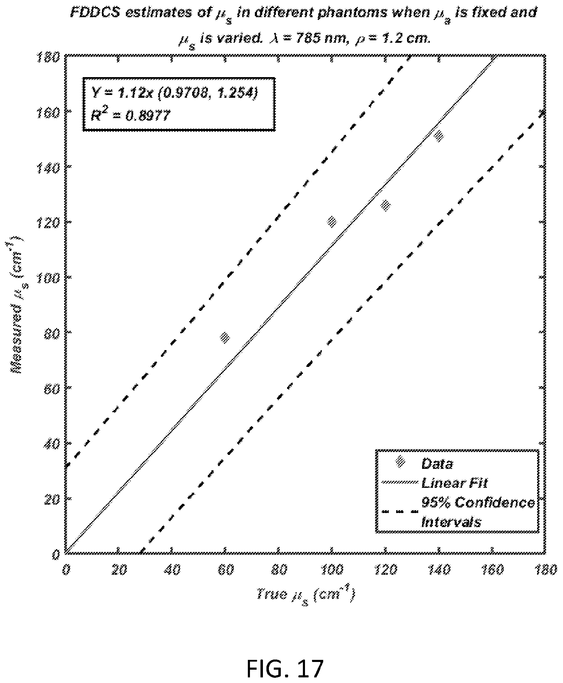

FIG. 17 shows an example plot of sensitivity to scattering changes in tissue simulating phantoms when scattering coefficient is varied and absorption coefficient is kept constant of a frequency domain diffuse correlation spectroscopy system implemented in accordance with some embodiments of the disclosed subject matter.

FIG. 17 demonstrates the sensitivity of FD-DCS system to scattering changes. Here, the scattering coefficient ($\mu_s'$) estimated with FD-DCS is on the y-axis, with the theoretical values along the x-axis. $\mu_a$ and $F$ were fixed for these phantoms. The solid diamond red markers indicate the estimated absorption coefficient, while the blue line is a linear fit to the data. The dashed black lines indicate the 95% confidence bounds of the linear regression. The slope of the regression line is 1.12 ($R^2 > 0.89$) indicating good 1:1 correspondence between measured and actual values of absorption coefficients. The results show that FD-DCS estimates of $\mu_s$ are linear.

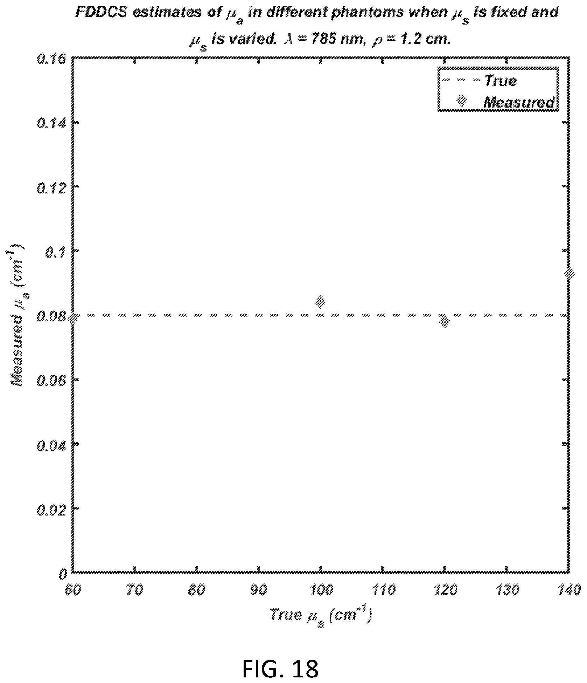

FIG. 18 shows an example plot of sensitivity to absorption changes in tissue simulating phantoms when scattering coefficient is varied and absorption coefficient is kept constant of a frequency domain diffuse correlation spectroscopy system implemented in accordance with some embodiments of the disclosed subject matter.

FIG. 18 shows the estimates of $\mu_a$ by FD-DCS system for different phantoms when $\mu_s$ is varied and $\mu_a$ is fixed.

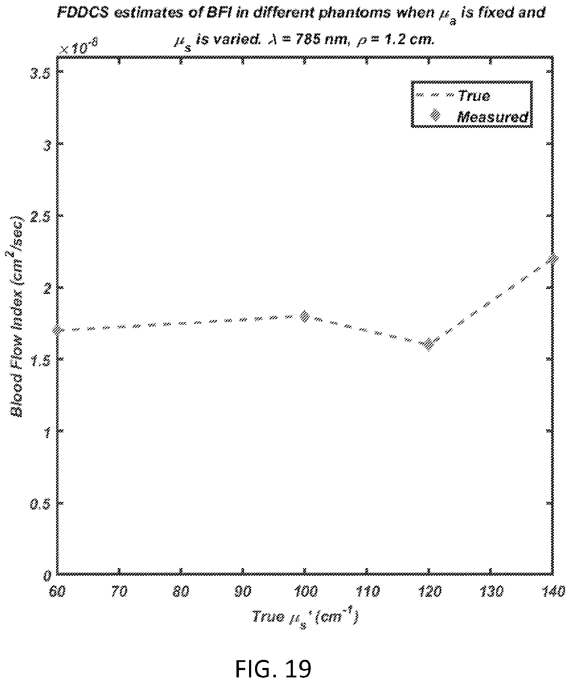

FIG. 19 shows an example plot of sensitivity to BFI changes in tissue simulating phantoms when scattering coefficient is varied and absorption coefficient is kept constant of a frequency domain diffuse correlation spectroscopy system implemented in accordance with some embodiments of the disclosed subject matter.

FIG. 19 shows the estimates of BFI by FD-DCS system for different phantoms when $\mu_s$ is varied and $\mu_a$ is fixed. The blood flow index are also in the order of 1e-8 and scattering coefficient for various phantoms in these phantoms were relatively constant.

In some embodiments, any suitable computer readable media can be used for storing instructions for performing the functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, any other suitable media that is fleeting and devoid of any semblance of permanence during transmission, and/or any suitable intangible media.

Various features and advantages of the invention are set forth in the following claims.

**Claims**

1. A system for frequency domain diffuse correlation spectroscopy (300), comprising:

    an intensity modulated coherent light source (304);
    a photon counting detector (306);
    at least one hardware processor (316) that is programmed to:

        cause the light source to emit light at a plurality of different intensity modulation frequencies toward a tissue sample (324);
        detect, for each of the plurality of different modulation frequencies, photon counts over a predetermined period of time;
        determine, for each of the plurality of modulation frequencies, a normalized intensity auto-correlation function;
        estimate a plurality of properties of the tissue sample using the plurality of intensity auto-correlation functions; and
        output at least one of the plurality of properties.

2. The system of claim 1, wherein the intensity modulated coherent light source comprises:

    a laser; and
    a signal generator.

3. The system of claim 1, wherein the photon counting detector comprises an avalanche photodiode.

4. The system of claim 1, wherein the plurality of different modulation frequencies comprises at least one frequency in a range of 50 megahertz (MHz) and 600 MHz.

5. The system of claim 1, wherein the intensity auto-correlation function is determined based on an equation expressed as:

$$g_2(\tau) = \frac{\langle I(t)I(t+\tau)\rangle_t}{\langle I(t)\rangle^2}$$

where $I(t)$ is intensity at the photon counting detector at time t, and $I(t + \tau)$ is intensity at the photon counting detector at time $t + \tau$.

6. The system of claim 5, wherein the normalized intensity auto-correlation function is modeled based on an equation expressed as:

$$g_2(\tau, \rho, \omega) = 1 + \beta[(1-m)|g_1(\rho, \tau, \omega = 0)|^2 + (m)|g_1(\rho, \tau, \omega = 0)|^2]$$

where $\beta$ is a speckle averaging factor, $m$ is modulation depth, and $g_1(\rho, \tau, \omega = 0)$ is a normalized electric field auto-correlation function; and
wherein the at least one hardware processor that is further programmed to:
estimate at least a subset of the plurality of properties of the tissue sample using the plurality of intensity auto-correlation functions based on the modeling of the normalized intensity auto-correlation function.

7. The system of claim 5, wherein the normalized intensity auto-correlation function is modeled based on an equation expressed as:

$$g_{2_{FD}}(\rho, \tau, \omega) = 1 + \beta \left[ \frac{|g_{1dc}(\tau)|^2 + 2m|g_{1dc}||g_{1ac}| + m^2|g_{1ac}(\tau)|^2}{(1+m)^2} \right]$$

where $\beta$ is a speckle averaging factor, m is modulation depth, and $g_{1dc}(\tau)$ and $g_{1ac}(\tau)$ are normalized electric field auto-correlation functions; and
wherein the at least one hardware processor that is further programmed to:
estimate at least a subset of the plurality of properties of the tissue sample using the plurality of intensity auto-correlation functions based on the modeling of the normalized intensity auto-correlation function.

8. The system of claim 1, wherein the plurality of properties comprises:

a tissue blood flow index $F$;
an absorption coefficient $\mu_a$; and
a scattering coefficient $\mu'_s$; and
further wherein the plurality of properties are indicative of one or more of:

presence of oxy-hemoglobin in the tissue sample;
presence of deoxy-hemoglobin in the tissue sample;
tissue oxygen saturation of the tissue sample; or
tissue metabolism rate of oxygen of the tissue sample.

9. A method for frequency domain diffuse correlation spectroscopy, comprising:

causing an intensity modulated coherent light source to emit light at a plurality of different intensity modulation frequencies toward a tissue sample (1102);
detecting, for each of the plurality of different modulation frequencies, photon counts over a predetermined period of time using a photon counting detector (1104);
determining, for each of the plurality of modulation frequencies, a normalized intensity auto-correlation function (1106);
estimating a plurality of properties of the tissue sample using the plurality of intensity auto-correlation functions (1108); and
outputting at least one of the plurality of properties (1110).

10. The method of claim 9, wherein,

the intensity modulated coherent light source comprises:

a laser; and
a signal generator; and

the photon counting detector comprises an avalanche photodiode

11. The method of claim 9, wherein the plurality of different modulation frequencies comprises at least one frequency in a range of 50 megahertz (MHz) and 600 MHz.

12. The method of claim 9, wherein the intensity auto-correlation function is determined based on an equation expressed as:

$$g_2(\tau) = \frac{\langle I(t)I(t+\tau)\rangle_t}{\langle I(t)\rangle^2}$$

where $I(t)$ is intensity at the photon counting detector at time t, and $I(t+\tau)$ is intensity at the photon counting detector at time $t + \tau$.

13. The method of claim 12, wherein the normalized intensity auto-correlation function is modeled based on an equation expressed as:

$$g_2(\tau, \rho, \omega) = 1 + \beta[(1 - m)|g_1(\rho, \tau, \omega = 0)|^2 + (m)|g_1(\rho, \tau, \omega = 0)|^2]$$

where $g_2(\tau, \rho, \omega)$ is the intensity auto-correlation function, $\beta$ is a speckle averaging factor, m is modulation depth, and $g_1(\rho, \tau, \omega = 0)$ is a normalized electric field auto-correlation function; and
wherein estimating the plurlaity of properties comprises:
estimating at least a subset of the plurality of properties of the tissue sample using the plurality of intensity auto-correlation functions based on the modeling of the normalized intensity auto-correlation function.

14. The method of claim 12, wherein the normalized intensity auto-correlation function is modeled based on an equation expressed as:

$$g_{2_{FD}}(\rho, \tau, \omega) = 1 + \beta\left[\frac{|g_{1dc}(\tau)|^2 + 2m|g_{1dc}||g_{1ac}| + m^2|g_{1ac}(\tau)|^2}{(1 + m)^2}\right]$$

where $\beta$ is a speckle averaging factor, $m$ is modulation depth, and $g_{1dc}(\tau)$ and $g_{1ac}(\tau)$ are normalized electric field auto-correlation functions; and
wherein the at least one hardware processor that is further programmed to:
estimate at least a subset of the plurality of properties of the tissue sample using the plurality of intensity auto-correlation functions based on the modeling of the normalized intensity auto-correlation function.

15. The method of claim 9, wherein the plurality of properties comprises:

a tissue blood flow index $F$;
an absorption coefficient $\mu_a$; and
a scattering coefficient $\mu'_s$; and
further wherein the plurality of properties are indicative of one or more of:

presence of oxy-hemoglobin in the tissue sample;
presence of deoxy-hemoglobin in the tissue sample;
tissue oxygen saturation of the tissue sample; or

tissue metabolism rate of oxygen of the tissue sample.

**Patentansprüche**

1. System für die Frequenzdomänen-Diffuskorrelationsspektroskopie (300), umfassend:

eine intensitätsmodulierte kohärente Lichtquelle (304);
einen Photonenzähldetektor (306);
mindestens einen Hardware-Prozessor (316), der programmiert ist zum:

Bewirken, dass die Lichtquelle Licht mit einer Vielzahl von unterschiedlichen Intensitätsmodulationsfrequenzen in Richtung einer Gewebeprobe (324) emittiert;
Erfassen, für jede der Vielzahl von unterschiedlichen Modulationsfrequenzen, der Photonenzahl über einen vorbestimmten Zeitraum hinweg;
Bestimmen, für jede der Vielzahl von Modulationsfrequenzen, einer normalisierte Intensitätsautokorrelationsfunktion;
Schätzen einer Vielzahl von Eigenschaften der Gewebeprobe unter Verwendung der Vielzahl von Intensitätsautokorrelationsfunktionen; und
Ausgeben mindestens einer der Vielzahl von Eigenschaften.

2. System nach Anspruch 1, wobei die intensitätsmodulierte kohärente Lichtquelle Folgendes umfasst:

einen Laser; und
einen Signalgenerator.

3. System nach Anspruch 1, wobei der Photonenzähldetektor eine Lawinenphotodiode umfasst.

4. System nach Anspruch 1, wobei die Vielzahl von verschiedenen Modulationsfrequenzen mindestens eine Frequenz in einem Bereich von 50 Megahertz (MHz) und 600 MHz umfasst.

5. System nach Anspruch 1, wobei die Intensitätsautokorrelationsfunktion auf der Grundlage einer Gleichung bestimmt wird, die wie folgt ausgedrückt wird:

$$g_2(\tau) = \frac{\langle I(t)I(t+\tau)\rangle_t}{\langle I(t)\rangle^2}$$

wobei $I(t)$ die Intensität am Photonenzähldetektor zum Zeitpunkt t ist und $I(t+\tau)$ die Intensität am Photonenzähldetektor zum Zeitpunkt $t + \tau$ ist.

6. System nach Anspruch 5, wobei die normalisierte Intensitätsautokorrelationsfunktion auf der Grundlage einer Gleichung modelliert wird, die wie folgt ausgedrückt wird:

$$g_2(\tau, \rho, \omega) = 1 + \beta[(1-m)|g_1(\rho, \tau, \omega = 0)|^2 + (m)|g_1(\rho, \tau, \omega = 0)|^2]$$

wobei $\beta$ ein Speckle-Mittelungsfaktor ist, $m$ die Modulationstiefe ist und $g_1(\rho,\tau,\omega = 0)$ eine normalisierte Autokorrelationsfunktion des elektrischen Feldes ist; und
wobei der mindestens eine Hardwareprozessor ferner programmiert ist zum:
Schätzen mindestens einer Teilmenge der Vielzahl von Eigenschaften der Gewebeprobe unter Verwendung der Vielzahl von Intensitätsautokorrelationsfunktionen auf der Grundlage der Modellierung der normalisierten Intensitätsautokorrelationsfunktion.

7. System nach Anspruch 5, wobei die normalisierte Intensitätsautokorrelationsfunktion auf der Grundlage einer Gleichung modelliert wird, die wie folgt ausgedrückt wird:

$$g_{2_{FD}}(\rho, \tau, \omega) = 1 + \beta \left[ \frac{|g_{1dc}(\tau)|^2 + 2m|g_{1dc}||g_{1ac}| + m^2|g_{1ac}(\tau)|^2}{(1+m)^2} \right]$$

wobei $\beta$ ein Speckle-Mittelungsfaktor ist, m die Modulationstiefe ist und $g_{1dc}(\tau)$ und $g_{1ac}(\tau)$ normalisierte Autokorrelationsfunktion des elektrischen Feldes sind; und
wobei der mindestens eine Hardwareprozessor ferner programmiert ist zum:
Schätzen mindestens einer Teilmenge der Vielzahl von Eigenschaften der Gewebeprobe unter Verwendung der Vielzahl von Intensitätsautokorrelationsfunktionen auf der Grundlage der Modellierung der normalisierten Intensitätsautokorrelationsfunktion.

8. System nach Anspruch 1, wobei die Vielzahl von Eigenschaften Folgendes umfasst:

einen Gewebeblutströmungsindex $F$;
einen Absorptionskoeffizienten $\mu_a$; und
einen Streukoeffizienten $\mu'_s$; und
wobei ferner die Vielzahl von Eigenschaften auf eines oder mehrere der folgenden Merkmale hinweist:

das Vorhandensein von Oxyhämoglobin in der Gewebeprobe;
das Vorhandensein von Desoxyhämoglobin in der Gewebeprobe;
die Gewebesauerstoffsättigung der Gewebeprobe; oder
die Sauerstoffstoffwechselrate des Gewebes der Gewebeprobe.

9. Verfahren zur Frequenzdomänen-Diffuskorrelationsspektroskopie, umfassend:

Bewirken, dass eine intensitätsmodulierte kohärente Lichtquelle Licht mit einer Vielzahl von verschiedenen Intensitätsmodulationsfrequenzen in Richtung einer Gewebeprobe (1102) emittiert;

Erfassen, für jede der Vielzahl von unterschiedlichen Modulationsfrequenzen, der Photonenzahl über einen vorbestimmten Zeitraum hinweg unter Verwendung eines Photonenzähldetektors (1104);

Bestimmen, für jede der Vielzahl von Modulationsfrequenzen, einer normalisierten Intensitätsautokorrelationsfunktion (1106);

Schätzen einer Vielzahl von Eigenschaften der Gewebeprobe unter Verwendung der Vielzahl von Intensitätsautokorrelationsfunktionen (1108); und

Ausgeben mindestens einer der Vielzahl von Eigenschaften (1110).

**10.** Verfahren nach Anspruch 9, wobei

die intensitätsmodulierte kohärente Lichtquelle Folgendes umfasst:

einen Laser; und
einen Signalgenerator; und

der Photonenzähldetektor eine Lawinenphotodiode umfasst.

**11.** Verfahren nach Anspruch 9, wobei die Vielzahl von verschiedenen Modulationsfrequenzen mindestens eine Frequenz in einem Bereich von 50 Megahertz (MHz) und 600 MHz umfasst.

**12.** Verfahren nach Anspruch 9, wobei die Intensitätsautokorrelationsfunktion auf der Grundlage einer Gleichung bestimmt wird, die wie folgt ausgedrückt wird:

$$g_2(\tau) = \frac{\langle I(t)I(t+\tau)\rangle_t}{\langle I(t)\rangle^2}$$

wobei $I(t)$ die Intensität am Photonenzähldetektor zum Zeitpunkt t ist und $I(t+\tau)$ die Intensität am Photonenzähldetek-tor zum Zeitpunkt $t + \tau$ ist.

**13.** Verfahren nach Anspruch 12, wobei die normalisierte Intensitätsautokorrelationsfunktion auf der Grundlage einer Gleichung modelliert wird, die wie folgt ausgedrückt wird:

$$g_2(\tau,\rho,\omega) = 1 + \beta[(1-m)|g_1(\rho,\tau,\omega=0)|^2 + (m)|g_1(\rho,\tau,\omega=0)|^2]$$

wobei $g_2(\tau,\rho,\omega)$ die Intensitätsautokorrelationsfunktion ist, $\beta$ ein Speckle-Mittelungsfaktor ist, $m$ die Modulations-tiefe ist und $g_1(\rho,\tau,\omega=0)$ eine Autokorrelationsfunktion des elektrischen Feldes ist; und
wobei das Schätzen der Vielzahl von Eigenschaften Folgendes umfasst:
Schätzen mindestens einer Teilmenge der Vielzahl von Eigenschaften der Gewebeprobe unter Verwendung der Vielzahl von Intensitätsautokorrelationsfunktionen auf der Grundlage der Modellierung der normalisierten Intensitätsautokorrelationsfunktion.

**14.** Verfahren nach Anspruch 12, wobei die normalisierte Intensitätsautokorrelationsfunktion auf der Grundlage einer Gleichung modelliert wird, die wie folgt ausgedrückt wird:

$$g_{2_{FD}}(\rho,\tau,\omega) = 1 + \beta\left[\frac{|g_{1dc}(\tau)|^2 + 2m|g_{1dc}||g_{1ac}| + m^2|g_{1ac}(\tau)|^2}{(1+m)^2}\right]$$

wobei $\beta$ ein Speckle-Mittelungsfaktor ist, $m$ die Modulationstiefe ist und $g_{1dc}(\tau)$ und $g_{1ac}(\tau)$ normalisierte Autokorrelationsfunktionen des elektrischen Feldes sind; und
wobei der mindestens eine Hardwareprozessor ferner programmiert ist zum:
Schätzen mindestens einer Teilmenge der Vielzahl von Eigenschaften der Gewebeprobe unter Verwendung der Vielzahl von Intensitätsautokorrelationsfunktionen auf der Grundlage der Modellierung der normalisierten Intensitätsautokorrelationsfunktion.

**15.** Verfahren nach Anspruch 9, wobei die Vielzahl von Eigenschaften Folgendes umfasst:

einen Gewebeblutströmungsindex *F*;
einen Absorptionskoeffizienten $\mu_a$; und
einen Streukoeffizienten $\mu'_s$; und
wobei ferner die Vielzahl von Eigenschaften auf eines oder mehrere der folgenden Merkmale hinweist:

das Vorhandensein von Oxyhämoglobin in der Gewebeprobe;
das Vorhandensein von Desoxyhämoglobin in der Gewebeprobe;
die Gewebesauerstoffsättigung der Gewebeprobe; oder
die Sauerstoffstoffwechselrate des Gewebes der Gewebeprobe.

## Revendications

**1.** Système pour spectroscopie de corrélation diffuse de domaine fréquentiel (300), comprenant.

une source de lumière cohérente à modulation d'intensité (304) ;
un détecteur de comptage de photon (306) ;
au moins un processeur matériel (316) qui est programmé pour
amener la source de lumière à émettre de la lumière à une pluralité de différentes fréquences de modulation d'intensité vers un échantillon tissulaire (324) ;
détecter, pour chacune de la pluralité de différentes fréquences de modulation, des comptes de photon au cours d'un laps de temps prédéterminé ;
déterminer, pour chacune de la pluralité de fréquences de modulation, une fonction d'autocorrélation d'intensité normalisée ;
estimer une pluralité de propriétés de l'échantillon tissulaire en utilisant la pluralité de fonctions d'autocorrélation d'intensité ; et
produire en sortie au moins une de la pluralité de propriétés.

**2.** Système de la revendication 1, dans lequel la source de lumière cohérente à modulation d'intensité comprend :

un laser ; et
un générateur de signal.

**3.** Système de la revendication 1, dans lequel le détecteur de comptage de photon comprend une photodiode à avalanche.

**4.** Système de la revendication 1, dans lequel la pluralité de différentes fréquences de modulation comprend au moins une fréquence dans une plage de 50 mégahertz (MHz) et 600 MHz.

**5.** Système de la revendication 1, dans lequel la fonction d'autocorrélation d'intensité est déterminée sur la base d'une équation exprimée sous la forme :

$$g_2(\tau) = \frac{\langle I(t)I(t+\tau)\rangle_t}{\langle I(t)\rangle^2}$$

où *I(t)* est l'intensité au détecteur de comptage de photon au temps *t*, et *I(t + $\tau$)* est l'intensité au détecteur de comptage de photon au temps *t + $\tau$*.

**6.** Système de la revendication 5, dans lequel la fonction d'autocorrélation d'intensité normalisée est modélisée sur la base d'une équation exprimée sous la forme :

$$g_2(\tau, \rho, \omega) = 1 + \beta\left[(1-m)|g_1(\rho, \tau, \omega = 0)|^2 + (m)|g_1(\rho, \tau, \omega = 0)|^2\right]$$

où $\beta$ est un facteur de moyenne de tacheture, $m$ est une profondeur de modulation, et $g_1(\rho, \tau, \omega = 0)$ est une fonction d'autocorrélation de champ électrique normalisée ; et

dans lequel l'au moins un processeur matériel est en outre programmé pour :

estimer au moins un sous-ensemble de la pluralité de propriétés de l'échantillon tissulaire en utilisant la pluralité de fonctions d'autocorrélation d'intensité sur la base de la modélisation de la fonction d'autocorrélation d'intensité normalisée.

7. Système de la revendication 5, dans lequel la fonction d'autocorrélation d'intensité normalisée est modélisée sur la base d'une équation exprimée sous la forme :

$$g_{2FD}(\rho, \tau, \omega) = 1 + \beta \left[ \frac{|g_{1dc}(\tau)|^2 + 2m|g_{1dc}||g_{1ac}| + m^2 |g_{1ac}(\tau)|^2}{(1+m)^2} \right]$$

où $\beta$ est un facteur de moyenne de tacheture, $m$ est une profondeur de modulation, et $g_{1dc}(\tau)$ et $g_{1ac}(\tau)$ sont des fonctions d'autocorrélation de champ électrique normalisées ; et

dans lequel l'au moins un processeur matériel qui est en outre programmé pour :

estimer au moins un sous-ensemble de la pluralité de propriétés de l'échantillon tissulaire en utilisant la pluralité de fonctions d'autocorrélation d'intensité sur la base de la modélisation de la fonction d'autocorrélation d'intensité normalisée.

8. Système de la revendication 1, dans lequel la pluralité de propriétés comprend :

un indice de débit sanguin tissulaire $F$ ;
un coefficient d'absorption $\mu_\alpha$ ; et
un coefficient de dispersion $\mu'_s$ ; et
en outre dans lequel la pluralité de propriétés sont indicatives d'un ou de plusieurs paramètres parmi :

la présence d'oxyhémoglobine dans l'échantillon tissulaire ;
la présence de désoxyhémoglobine dans l'échantillon tissulaire ;
la saturation d'oxygène tissulaire de l'échantillon tissulaire ; ou
le taux métabolique tissulaire de l'échantillon tissulaire.

9. Procédé pour spectroscopie de corrélation diffuse de domaine fréquentiel, comprenant les faits suivants :

amener une source de lumière cohérente à modulation d'intensité à émettre de la lumière à une pluralité de différentes fréquences de modulation d'intensité vers un échantillon tissulaire (1102) ;
détecter, pour chacune de la pluralité de différent fréquences de modulation, des comptes de photon au cours d'un laps de temps prédéterminé en utilisant un détecteur de comptage de photon (1104) ;
déterminer, pour chacune de la pluralité de fréquences de modulation, une fonction d'autocorrélation d'intensité normalisée (1106) ;
estimer une pluralité de propriétés de l'échantillon tissulaire en utilisant la pluralité de fonctions d'autocorrélation d'intensité (1108) ; et
produire en sortie au moins une de la pluralité de propriétés ( 1110).

10. Procédé de la revendication 9, dans lequel la source de lumière cohérente à modulation d'intensité comprend :

un laser ; et
un générateur de signal ; et
le détecteur de comptage de photon comprend une photodiode à avalanche

11. Procédé de la revendication 9, dans lequel la pluralité de différentes fréquences de modulation comprend au moins une fréquence dans une plage de 50 mégahertz (MHz) et 600 MHz.

12. Procédé de la revendication 9, dans lequel la fonction d'autocorrélation d'intensité est déterminée sur la base d'une équation exprimée sous la forme :

$$g_2(\tau) = \frac{\langle I(t)I(t+\tau)\rangle_t}{\langle I(t)\rangle^2}$$

où $I(t)$ est l'intensité au détecteur de comptage de photon au temps $t$, et $I(t+\tau)$ est l'intensité au détecteur de comptage de photon au temps $t + \tau$.

**13.** Procédé de la revendication 12, dans lequel la fonction d'autocorrélation d'intensité normalisée est modélisée sur la base d'une équation exprimée sous la forme :

$$g_2(\tau, \rho, \omega) = 1 + \beta\left[(1-m)|g_1(\rho, \tau, \omega = 0)|^2 + (m)|g_1(\rho, \tau, \omega = 0)|^2\right]$$

où $g_2(\tau, \rho, \omega)$ est la fonction d'autocorrélation d'intensité, $\beta$ est un facteur de moyenne de tacheture, $m$ est une profondeur de modulation, et $g_1(\rho, \tau, \omega = 0)$ est une fonction d'autocorrélation de champ électrique normalisée ; et dans lequel le fait d'estimer la pluralité de propriétés comprend le fait suivant :
estimer au moins un sous-ensemble de la pluralité de propriétés de l'échantillon tissulaire en utilisant la pluralité de fonctions d'autocorrélation d'intensité sur la base de la modélisation de la fonction d'autocorrélation d'intensité normalisée.

**14.** Procédé de la revendication 12, dans lequel la fonction d'autocorrélation d'intensité normalisée est modélisée sur la base d'une équation exprimée sous la forme :

$$g_{2FD}(\rho, \tau, \omega) = 1 + \beta\left[\frac{|g_{1dc}(\tau)|^2 + 2m|g_{1dc}||g_{1ac}| + m^2|g_{1ac}(\tau)|^2}{(1+m)^2}\right]$$

où $\beta$ est un facteur de moyenne de tacheture, $m$ est une profondeur de modulation, et $g_{1dc}(\tau)$ et $g_{1ac}(\tau)$ sont des fonctions d'autocorrélation de champ électrique normalisées ; et
dans lequel l'au moins un processeur matériel qui est en outre programmé pour :
estimer au moins un sous-ensemble de la pluralité de propriétés de l'échantillon tissulaire en utilisant la pluralité de fonctions d'autocorrélation d'intensité sur la base de la modélisation de la fonction d'autocorrélation d'intensité normalisée.

**15.** Procédé de la revendication 9, dans lequel la pluralité de propriétés comprend :

    un indice de débit sanguin tissulaire $F$ ;
    un coefficient d'absorption $\mu_\alpha$ ; et
    un coefficient de dispersion $\mu'_s$ ; et
    en outre dans lequel la pluralité de propriétés sont indicatives d'un ou de plusieurs paramètres parmi :

        la présence d'oxyhémoglobine dans l'échantillon tissulaire ;
        la présence de désoxyhémoglobine dans l'échantillon tissulaire ;
        la saturation d'oxygène tissulaire de l'échantillon tissulaire ; ou
        le taux métabolique tissulaire de l'échantillon tissulaire.

COMMERCIAL STANDARD

functional Near
Infrared Spectroscopy
**(fNIRS)**

*Changes* in tissue oxygenation

*Special
case*

RESEARCH GRADE

Frequency Domain
Diffuse Optical
Spectroscopy (FD-DOS)

Quantitative tissue optical
properties, oxygenation

Diffuse Correlation
Spectroscopy (DCS)

Quantitative tissue perfusion

DESCRIBED HEREIN

Frequency Domain
Diffuse Correlation
Spectroscopy (FD-DCS)

Quantitative tissue optical prop.,
oxygenation & perfusion

FIG. 1

EP 4 447 797 B1

**A**

Before                                    After

Tissue
Absorber

Photon $\sim\!\!\sim\!\!\sim\!\!\sim\!\!\!\!-\mathbf{k}_i$ ◯                    ◯

**B**

Before                    After

Tissue
Scatterer

Photon $\sim\!\!\sim\!\!\sim\!\!\sim\!\!\!\!-\mathbf{k}_i$ ◯        ◯ $\sim\!\!\sim\!\!\sim\!\!\sim\!\!\!\mathbf{k}_s$

FIG. 2

EP 4 447 797 B1

300

EP 4 447 797 B1

FIG. 3

FIG. 4

EP 4 447 797 B1

FIG. 5

FIG. 6

FIG. 7

FIG. 8

FIG. 9

FIG. 10

1100

EMIT LONG-COHERENCE LASER WITH MODULATED AT MULTIPLE DIFFERENT MODULATION FREQUENCIES — 1102

DETECT, FOR EACH MODULATION FREQUENCY, VARIATIONS IN INTENSITY OF RECEIVED LIGHT OVER A PREDETERMINED PERIOD OF TIME USING A PHOTON COUNTING DETECTOR AT A PARTICULAR SAMPLING RATE — 1104

DETERMINE AN INTENSITY AUTO-CORRELATION FUNCTION FOR EACH MODULATION FREQUENCY — 1106

ESTIMATE FLOW F, ABSORPTION COEFFICIENT $\mu_a$, AND ABSORPTION COEFFICIENT $\mu'_s$ USING THE INTENSITY AUTO-CORRELATION FUNCTIONS — 1108

CAUSE A TISSUE PARAMETER(S) TO BE PRESENTED — 1110

FIG. 11

FIG. 12

EP 4 447 797 B1

FIG. 13

EP 4 447 797 B1

FDDCS estimates of $\mu_a$ in different phantoms when $\mu_s$ is fixed and $\mu_a$ is varied. $\lambda = 785$ nm, $\rho = 1.2$ cm.

$Y = 1.05x \ (0.7921, 1.308)$
$R^2 = 0.8168$

Data
Linear Fit
95% Confidence Intervals

Measured $\mu_a$ (cm$^{-1}$)

True $\mu_a$ (cm$^{-1}$)

FIG. 14

FIG. 15

FIG. 16

FIG. 17

FIG. 18

EP 4 447 797 B1

FIG. 19

EP 4 447 797 B1

**EP 4 447 797 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63265626
- US 2019000321 A
- US 2019261869 A

**Non-patent literature cited in the description**

- **APPLEGATE MATTHEW B. et al.** Modulation frequency selection and efficient look-up table inversion for frequency domain diffuse optical spectroscopy. *JOURNAL OF BIOMEDICAL OPTICS*, 2021